(19) Europäisches Patentamt
European Patent Office
Office européen des brevets

(11) EP 1 920 762 A1

(12) **EUROPEAN PATENT APPLICATION**
published in accordance with Art. 153(4) EPC

(43) Date of publication:
**14.05.2008 Bulletin 2008/20**

(21) Application number: **06781633.0**

(22) Date of filing: **26.07.2006**

(51) Int Cl.:
***A61K 8/86** (2006.01)* ***A61K 8/06** (2006.01)*
***A61Q 17/04** (2006.01)*

(86) International application number:
**PCT/JP2006/314721**

(87) International publication number:
**WO 2007/013484 (01.02.2007 Gazette 2007/05)**

(84) Designated Contracting States:
**DE ES FR GB IT**

(30) Priority: **26.07.2005 JP 2005216492**
**26.07.2005 JP 2005216493**
**10.08.2005 JP 2005232553**
**10.08.2005 JP 2005232554**

(71) Applicant: **Shiseido Company, Limited**
**Chuo-ku**
**Tokyo 104-8010 (JP)**

(72) Inventors:
- **KAKOKI, Hiroyuki**
**suzuki-ku,Yokohama-shi, Kanagawa 2248558 (JP)**
- **OHMORI, Takashi**
**chome, Tsuzuki-ku, Yokohama-shi, Kanagawa 2248558 (JP)**
- **NASU, Akio**
**chome, Tsuzuki-ku, Yokohama-shi, Kanagawa 2248558 (JP)**
- **ISHIKUBO, Akira**
**chome, Tsuzuki-ku,Yokohama-shi, Kanagawa 2248558 (JP)**

(74) Representative: **Henkel, Feiler & Hänzel**
**Patentanwälte**
**Maximiliansplatz 21**
**80333 München (DE)**

# (54) EMULSION-TYPE EXTERNAL PREPARATIONS FOR SKIN

(57) The emulsified external skin preparation of the present invention is **characterized by** comprising a polyglycerol derivative represented by formula (1):

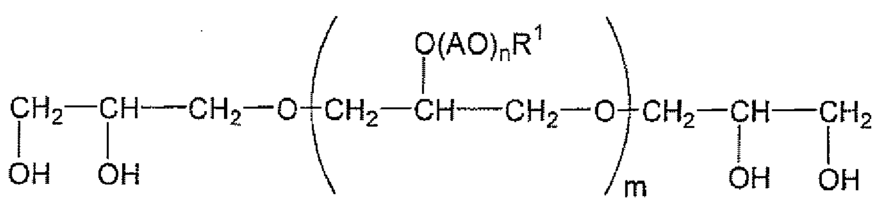

(1)

wherein, m + 2 represents the average polymerization degree of polyglycerol and 1≦m≦4;
$R^1$ is a hydrocarbon group having 1 to 4 carbon atoms or a hydrogen atom;
AO is an oxyalkylene group having 3 to 4 carbon atoms; and
n is the average addition mole number of the oxyalkylene group and 1≦m×n≦200. In a water-in-oil type external skin preparation, and especially in that containing a polar oil or a silicone oil, emulsion stability is improved. In an oil-in-water type external skin preparation, feeling in use and emulsion stability are improved. Especially, in an oil-in-water type external skin preparation containing hydrophobized powder, powder dispersion stability is improved as well as feeling in use and emulsion stability.

EP 1 920 762 A1

## Description

## RELATED APPLICATIONS

This application claims the priority of Japanese Patent Application Nos. 2005-216492 and 2005-216493 filed on July 26, 2005 and Japanese Patent Application Nos. 2005-232553 and 2005-232554 filed on August 10, 2005, which are incorporated herein by reference.

## FIELD OF THE INVENTION

The present invention relates to the improvement of the stability and the improvement of the feeling in use of emulsified external skin preparations. For example, the invention relates to the improvement of the emulsion stability of water-in-oil type external skin preparations, and in particular, of those containing polar oil or silicone oil. In addition, the invention relates to the improvement of the powder dispersion, emulsion stability, and feeling in use of oil-in-water type external skin preparations, and in particular, of those containing hydrophobized powder.

## BACKGROUND OF THE INVENTION

In the past, emulsion compositions have been widely used as external skin preparations; however, the improvement of the stability and the improvement of feeling in use have been desired.
For example, in the preparation of water-in-oil type cream, milky lotion, etc. containing nonpolar oil components such as liquid paraffin and squalane, nonionic surfactants with low HLB have been used as an emulsifier. However, there has been a problem in that if polar oils such as triglycerides or ester oils are blended, the system becomes unstable.
In recent years, numerous sunscreen cosmetics containing an UV absorber have been developed because people are increasingly conscious about the protection of skin from UV light. As the oil components to dissolve UV absorbers, it is necessary to blend polar oils such as triglycerides and ester oils. In addition, it is necessary to blend silicone oils such as cyclomethicone and dimethylpolysiloxane to improve the feeling in use. Therefore, water-in-oil type external skin preparations in which various oil components such as polar oils and silicone oils can be stably blended, in addition to nonpolar oils, have been demanded.
In order to solve these problems, polyolpolyhydroxystearate having 2 to 20 self-condensed polyhydroxystearic acids as a lipophilic group (refer to patent literature 1, for example) and A-B-A type block copolymers having an oligomer of hydroxycarboxylic acid as a lipophilic group and polyoxyalkylene as a hydrophilic group have been reported (refer to patent literature 2, for example). However, there have been issues in that the stability of these is poor at a low temperature and that they are not suitable to the emulsification of silicone oil.
As described above, the conventional water-in-oil type surfactants are not suitable to the emulsification with the use of polar oils or silicone oils, and the range of desired water-in-oil type external skin preparations has been limited. Thus, there has been a limit in the feeling in use, and there have been issues such as stickiness and poor spreadability. In addition, there has been an issue in that the blending of UV absorbers, which are hardly soluble in nonpolar oil components, is difficult.
In the past, various external skin preparations in which inorganic powders such as titanium oxide and zinc oxide are blended, have been widely used. As the base of these external skin preparations, a water-in-oil type emulsion base or powder base have mainly be used. These bases often do not provide a good feeling in use compared with oil-in-water type emulsion bases because of their strong oily or powdery feeling. In contrast, oil-in-water type emulsion bases are used for cosmetics such as milky lotion, cream, and emulsion-type foundation because they provide a fresh feeling in use.
However, in the case of oil-in-water type emulsion bases containing inorganic powder, there has been an issue in that powders tend to aggregate, and it was necessary to uniformly disperse powders. For example, in the case of sunscreen cosmetics, which provide an UV protection function, there have been various problems, if powders stay aggregated, such as a lowered UV protection effect or a changed feeling in use. Thus, a technology has been developed in that hydrophobized powders obtained by hydrophobizing the surface of inorganic powders such as titanium oxide and zinc oxide are blended in oil-in-water type emulsions. However, the dispersion of the hydrophobized powder and the emulsion stability have not been satisfactory. In addition, there has been a problem in that a sticky feeling is caused though a moist feeling could be achieved after use.
In order to solve these problems, techniques have been reported in which the dispersion stability is provided by the prevention of coalescence of emulsified particles and the prevention of aggregation and sedimentation of fine powder particles, which are caused because of time and temperature change (refer to patent literatures 3 and 4, for example). Even with these techniques, the dispersion stability of the hydrophobized powder, the emulsion stability, and the feeling in use were still not satisfactory. In particular, it is desirable to blend a large amount of hydrophobized

particulate titanium dioxide in sunscreen cosmetics etc. because of its UV screening ability. However, when a large amount of the hydrophobized particulate titanium dioxide was added, it was very difficult to satisfy the dispersion stability and the emulsion stability.

In recent years, a technique is sought-after in which both hydrophobized titanium oxide, which is mainly effective against UV-B, and hydrophobized zinc oxide, which is mainly effective against UV-A, are blended in a formulation of sunscreen cosmetics in order to provide an excellent UV screening ability. However, it has been very difficult to satisfy good dispersion and emulsion stability because the significant aggregation and coalescence of emulsified particles take place due to the coexistence of titanium oxide and zinc oxide, which surface states are different from each other.

Patent literature 1: PCT Japanese Translation Publication No. H10-501252
Patent literature 2: Japanese Unexamined Patent Publication No. H11-12125
Patent literature 3: Japanese Examined Patent Publication No. H07-94366
Patent literature 4: Japanese Unexamined Patent Publication No. H08-310940

## DISCLOSURE OF THE INVENTION

## PROBLEM TO BE SOLVED BY THE INVENTION

The present invention was made in view of the above-described problems of the conventional technique, and an object thereof is to provide water-in-oil type external skin preparations wherein the emulsion stability is excellent, and in particular, the emulsion stability is improved when polar oils or silicone oils are blended.
Another object of the present invention is to provide oil-in-water type external skin preparations wherein the feeling in use and the emulsion stability are excellent, and in particular, the feeling in use, dispersion stability, and emulsion stability are improved when hydrophobized powder is blended.

## MEANS TO SOLVE THE PROBLEM

The present inventors have diligently studied to achieve the above-described objects. As a result, the present inventors have found that the above-described problems could be solved by blending a polyglycerol derivative of a specific structure into emulsified external skin preparations.
More specifically, the present inventors have found that by blending a polyglycerol derivative of a specific structure as an emulsifier for a water-in-oil type external skin preparation, a water-in-oil type external skin preparation excellent in emulsion stability, compared with the cases in which a conventional common lipophilic surfactant is used, could be obtained. In particular, the emulsion stability are significantly improved in the case of the water-in-oil type external skin preparation containing a polar oil or a silicone oil.

In addition, the present inventors have found that by blending a polyglycerol derivative of a specific structure, an oil-in-water type external skin preparation excellent in the feeling in use and the emulsion stability could be obtained. In particular, the powder dispersion stability and the emulsion stability are significantly improved in the case of the oil-in-water type external skin preparation containing hydrophobized powder.

In addition, the present inventors have found that by blending an inulin derivative of a specific structure, and/or a block-type alkylene oxide derivative of a specific structure as well as a polyglycerol derivative of a specific structure, an oil-in-water type external skin preparation containing hydrophobized powder, wherein the feeling in use, powder dispersion stability, and emulsion stability are excellent, could be obtained. In particular, the powder dispersion stability and the emulsion stability are significantly improved in the case of the oil-in-water type external skin preparation containing both hydrophobized titanium oxide and hydrophobized zinc oxide. Thus, the present invention has been accomplished.

The emulsified external skin preparation of the present invention is characterized by comprising a polyglycerol derivative represented by formula (1):

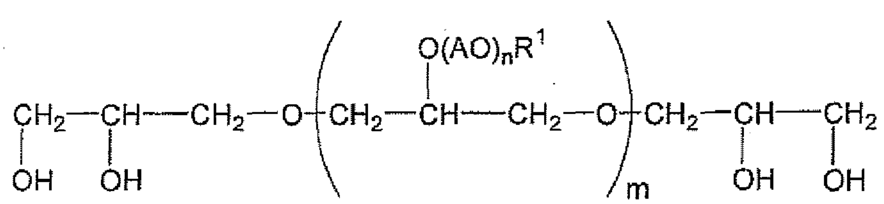

(1)

wherein, m + 2 represents the average polymerization degree of polyglycerol and $1 \leqq m \leqq 4$;

$R^1$ is a hydrocarbon group having 1 to 4 carbon atoms or a hydrogen atom;
AO is an oxyalkylene group having 3 to 4 carbon atoms; and
n is the average addition mole number of the oxyalkylene group and $1 \leqq m \times n \leqq 200$.

When the external skin preparation of the present invention is a water-in-oil type external skin preparation, it is preferable that the preparation further comprises a polar oil. In the water-in-oil type external skin preparation, it is preferable that the polar oil is one or more selected from the group consisting of polar oils with an IOB value of 0.05 to 0.80. In the water-in-oil type external skin preparation, it is preferable that the polar oil is one or more selected from the group consisting of 2-ethylhexyl p-methoxycinnamate, 2-ethylhexyl 2-cyano-3,3-diphenylacrylate, tripropylene glycol dipivalate, cetyl octanoate, trimethylolpropane tri-2-ethylhexanoate, pentaerythritol tetra-2-ethylhexanoate, glyceryl tri-2-ethylhexanoate, $C_{12-15}$ alkyl benzoate, caprylic/capric triglyceride, propylene glycol dicaprylate/dicaprate, and di-2-ethylhexyl succinate.
In the water-in-oil type external skin preparation, it is preferable that the preparation further comprises a silicone oil. In the water-in-oil type external skin preparation, it is preferable that the preparation further comprises an UV absorber that is a solid at ordinary temperature. In the water-in-oil type external skin preparation, it is preferable that the UV absorber that is a solid at ordinary temperature is selected from the group consisting of 2,4-bis-[[4-(2-ethylhexyloxy)-2-hydroxy]-phenyl]-6-(4-methoxyphenyl)-1,3,5-triazine, 2,4,6-trianilino-(p-carbo-2'-ethylhexyl-1'-oxy)-1,3,5-triazine, and 1-[4-(1,1-dimethylethyl) phenyl]-3-(4-methoxyphenyl)-1,3-propanedione. In the water-in-oil type external skin preparation, it is preferable that the preparation further comprises an UV scatterer. The water-in-oil type external skin preparation is preferably used as a sunscreen.
The water-in-oil type external skin preparation of the present invention contains the polyglycerol derivative of the specific structure as an emulsifier, thus it has excellent emulsion stability compared with the cases in which a conventional common lipophilic surfactant is used. In particular, the emulsion stability of the water-in-oil type external skin preparation containing a polar oil or a silicone oil can be significantly improved.
When the external skin preparation of the present invention is an oil-in-water type external skin preparation, it is preferable that the preparation further comprises hydrophobized powder. In the oil-in-water type external skin preparation, it is preferable that the hydrophobized powder is hydrophobized particulate titanium dioxide and/or hydrophobized particulate zinc oxide.
In the oil-in-water type external skin preparation containing hydrophobized powder of the present invention, it is preferable that the preparation further comprises an inulin derivative represented by formula (2-a) and/or a block-type alkylene oxide derivative represented by formula (2-b). Furthermore, it is preferable that the preparation comprises the hydrophobized particulate titanium dioxide and hydrophobized particulate zinc oxide as the hydrophobized powder.
$$A\text{-}(O\text{-}CO\text{-}NH\text{-}R^1)s \qquad (2\text{-}a)$$

wherein A is a fructose residue of inulin;

(O-CO-NH-$R^1$) represents a N-alkylaminocarbonyloxy group substituting a hydroxyl group of the fructose;
$R^1$ is a hydrocarbon group having 3 to 22 carbon atoms; and
s is the substitution degree of the N-alkylaminocarbonyloxy group per fructose residue, and s takes 0.10 to 2.0.

$$R^1O\text{-}(AO)_m(EO)_n\text{-}R^2 \qquad (2\text{-}b)$$

wherein AO is an oxyalkylene group having 3 to 4 carbon atoms;

EO is an oxyethylene group;
m and n are average addition mole numbers of the oxyalkylene group and the oxyethylene group respectively, which are $1 \leqq m \leqq 70, 1 \leqq n \leqq 70$;
the oxyalkylene group having 3 to 4 carbon atoms and the oxyethylene group are added to each other in block form;
the oxyethylene group is 20 to 80 mass % with respect to the sum of the oxyalkylene group having 3 to 4 carbon atoms and the oxyethylene group; and
$R^1$ and $R^2$ are either identical to or different from each other, and they are either a hydrocarbon group having 1 to 4 carbon atoms or hydrogen atom, and wherein the ratio of the number of the hydrogen atom with respect to the number of the hydrocarbon group in $R^1$ and $R^2$ is 0.15 or less.

In the oil-in-water type external skin preparation of the present invention, it is preferable that the preparation further comprises one or more selected from the group consisting of succinoglycan, xanthan gum, and acrylamide. In the oil-in-water type external skin preparation, it is preferable that the preparation further comprises one or more selected from the group consisting of carboxymethylcellulose, hydroxyethylcellulose, hydroxymethylcellulose, and gelatin. The oil-in-water type emulsified external skin preparation is preferably used as a sunscreen.

The oil-in-water type external skin preparation of the present invention contains the polyglycerol derivative of the specific structure, thus the feeling in use and the emulsion stability are excellent. In particular, the powder dispersion stability and the emulsion stability of the oil-in-water type external skin preparation containing hydrophobized powder can be significantly improved. When the oil-in-water type external skin preparation contains a inulin derivative of a specific structure and/or a block-type alkylene oxide derivative of a specific structure as well as the polyglycerol derivative of the specific structure, the oil-in-water type external skin preparation containing hydrophobized powder has excellent feeling in use, excellent powder dispersion stability, and excellent the emulsion stability. In particular, the powder dispersion stability and the emulsion stability of the preparation containing both hydrophobized titanium oxide and hydrophobized zinc oxide can be significantly improved.

## BEST MODE FOR CARRYING OUT THE INVENTION

The present invention will hereinafter be described in further detail with reference to specific examples. However, the present invention is not limited by these examples.

Polyglycerol derivatives

The polyglycerol derivative used in the present invention is represented by the below-described formula (1):

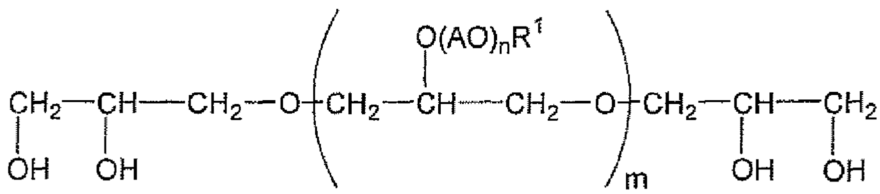

(1)

wherein, m + 2 represents the average polymerization degree of polyglycerol and $1 \leqq m \leqq 4$;

$R^1$ is a hydrocarbon group having 1 to 4 carbon atoms or a hydrogen atom;
AO is an oxyalkylene group having 3 to 4 carbon atoms; and
n is the average addition mole number of the oxyalkylene group and $1 \leqq m \times n \leqq 200$.

In the polyglycerol derivative represented by the above-described formula (1), m + 2 represents the average polymerization degree of polyglycerol, and $1 \leqq m \leqq 4$, namely, $3 \leqq m + 2 \leqq 6$. Examples of polyglycerols include triglycerol (m =1), tetraglycerol (m = 2), pentaglycerol (m = 3), and hexaglycerol (m = 4), and triglycerol is particularly preferable. When m is 0 (i.e., m + 2 = 2) or m is 5 or larger (i.e., $m + 2 \geqq 7$), the surface activity is not satisfactory.

A producing method of the polyglycerol used as the raw material for the preparation of the polyglycerol derivative of the present invention is not limited in particular, and the polyglycerol can be linear or branched. In the above-described formula (1), only linear polyglycerol derivative is shown for convenience. In the present invention, for example, it can be branched polyglycerol derivative, or a mixture thereof. Normally, commercial polyglycerols are obtained by dehydration condensation, in which the reaction can occur between position 1 or 3 and position 1 or 3, between position 1 or 3 and position 2, and between position 2 and position 2 of glycerol, resulting in a mixture of linear and branched polyglycerols. When such a mixture is used as the raw material, the polyglycerol derivative is obtained as a mixture of linear and branched polyglycerol derivatives.

Polyglycerol used as the raw material is preferably triglycerol with a narrow distribution of the average polymerization degree. The surface activity can be improved by using, as the raw material, triglycerol with its content of 75 mass % or more, and more preferably 80 mass % or more. The distribution of the average polymerization degree for triglycerol can be narrowed by distillation etc. The content of triglycerol can be measured, for example, by the following method.

Trimethylsilylation (TMS): Into a screw vial is weighed 0.1 g of a sample, and 0.5 mL of pyridine is added to

dissolve the sample. Subsequently, 0.4 mL of hexamethyldisilazane is added and mixed; then 0.2 mL of chlorotrimethylsilane is added and mixed well. The mixture was allowed to stand for 30 minutes, and then centrifuged to precipitate pyridine hydrochloride. The supernatant was filtered and analyzed by gas chromatography.

Detector: FID
Column: HP-5 Crosslinked 5% PH ME Siloxane, 0.25 μm × 30 m
Column temperature: 8D °C→320 °C (15 °C/min) 320 °C, 25 min
Inlet temperature: 320 °C
Detector temperature: 320 °C
Carrier gas: helium
Flow rate: 23 cm/sec
Injection volume: 0.2μL
Split ratio: splitless

$R^1$ is a hydrocarbon group having 1 to 4 carbon atoms or a hydrogen atom, and preferably a hydrocarbon group having 1 to 4 carbon atoms. Examples of hydrocarbon groups include saturated hydrocarbon groups such as methyl group, ethyl group, n-propyl group, isopropyl group, n-butyl group, s-butyl group, and t-butyl group; unsaturated hydrocarbon groups such as vinyl group and allyl group; and a mixture thereof, and a saturated hydrocarbon group is preferable.
AO is an oxyalkylene group having 3 to 4 carbon atoms, and the examples include oxypropylene group, oxybutylene group, oxyisobutylene group, oxy-t-butylene group, trimethylene group, tetramethylene group, and a mixture thereof. The percentage of oxybutylene group having 4 carbon atoms with respect to the total oxyalkylene groups is preferably 50 mass % or more, and more preferably 100 mass %. An oxyalkylene group having 2 or less carbon atoms is susceptible to salt concentration. In the case of an oxyalkylene group having 5 or more carbon atoms, it is difficult to obtain a high-purity derivative. The polymerization of different oxyalkylene groups can be either block-type or random-type.
The n represents the average addition mole number of oxyalkylene group. When it is expressed in combination with m, which represents the number of the hydroxyl group that can bond with the oxyalkylene group, $1 \leqq m \times n \leqq 200$, preferably $4 \leqq m \times n \leqq 100$, and more preferably $8 \leqq m \times n \leqq 70$. If $m \times n$ is zero, surface activity cannot be obtained. If $m \times n$ exceeds 200, it is difficult to obtain a high-purity derivative. The n, which represents the average addition mole number of the oxyalkylene group, can take the value $1 \leqq n \leqq 200$, preferably $4 \leqq n \leqq 80$, and more preferably $8 \leqq n \leqq 50$.
Specific examples of polyglycerol derivatives used in the present invention include polyoxybutylene(25 mol) methyl triglyceryl ether, polyoxybutylene(28 mol)methyl triglyceryl ether, polyoxybutylene(42 mol)methyl triglyceryl ether, polyoxybutylene(56 mol)methyl triglyceryl ether, polyoxybutylene(28 mol) triglyceryl ether, polyoxybutylene(42 mol) triglyceryl ether, polyoxybutylene(50 mol) triglyceryl ether, and polyoxybutylene(56 mol) triglyceryl ether.
The polyglycerol derivative represented by formula (1) of the present invention can be normally produced according to the following steps (1) to (3).

(1) Polyglycerol with an average polymerization degree of 3 to 6 is reacted with a ketalizing agent or acetalizing agent in the presence of an acid catalyst to obtain a diketalized polyglycerol or a diacetalized polyglycerol.
(2) Subsequently, an addition reaction of alkylene oxide having 3 to 4 carbon atoms is carried out in the presence of an alkaline catalyst. If necessary, the reaction with an alkyl (alkenyl) halide is further carried out in the presence of an alkaline catalyst, to achieve alkyl (alkenyl) etherification at the oxyalkylene terminal.
(3) Then deketalization or deacetalization is conducted in the presence of an acid catalyst.

The compound for ketalization or acetalization of polyglycerol is shown in formula (3):

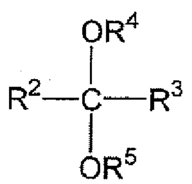

(3)

wherein $R^2$ and $R^3$ represent hydrocarbon groups having 1 to 4 carbon atoms or hydrogen atoms, respectively. $R^4$ and $R^5$ represent hydrocarbon groups having 1 to 4 carbon atoms, respectively. However, the case in which both

$R^2$ and $R^3$ are hydrogen atoms is excluded. Examples of hydrocarbon groups include methyl group, ethyl group, n-propyl group, isopropyl group, n-butyl group, s-butyl group, and t-butyl group, and methyl group is more preferable.

Examples of compounds represented by formula (3) include 2,2-dimethoxypropane, 1,1-dimethoxy-3-butanone, and 1,1-dimethoxyethane; and 2,2-dimethoxypropane is more preferably. Ketal compounds and acetal compounds can be directly synthesized from normal ketones or aldehydes. However, it is preferable to use the compound represented by formula (3) from the standpoint of the substitution reaction rate of the ketal group etc. Examples of ketalization or acetalization catalysts include acid catalysts such as sulfuric acid, and p-toluenesulfonic acid. Normally, the loaded amount of a compound represented by formula (3) is 250 to 500 mol % with respect to polyglycerol, and the loaded amount of an acid catalyst is 0.0005 to 0.015 mol % with respect to polyglycerol. The reaction temperature is generally 30 to 70 ˚C.

A diketalized polyglycerol produced by the reaction of polyglycerol with the compound of formula (3) is represented by the following formula (4):

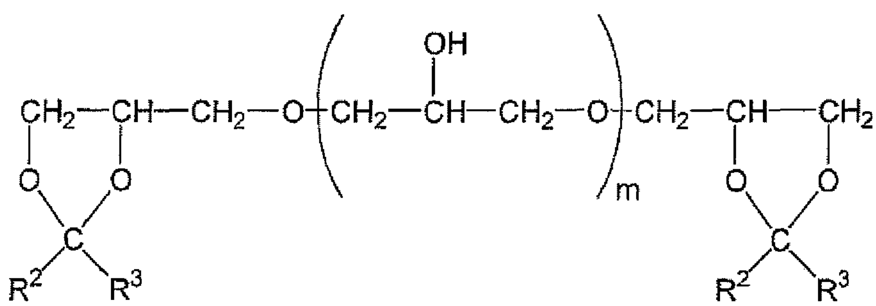

(4)

When the diketalized polyglycerol or diacetalized polyglycerol of formula (4) is used in the succeeding alkylene oxide addition reaction, the removal of catalysts is not particularly necessary. If necessary, however, neutralization with an alkali, acid adsorption, filtration, etc. can be conducted. For example, neutralizing agents such as sodium hydroxide, potassium hydroxide, sodium carbonate, and sodium acetate; adsorbents such as Kyowado 300 and Kyowado 1000 of Kyowa Chemical Industry Co., Ltd. and Tomix AD-500 of Tomita Pharmaceutical Co., Ltd.; and zeolites can be used.

The diketalized polyglycerol or diacetalized polyglycerol of formula (4) is described as a reaction product at positions 1 and 2 of the terminal glyceryl groups. However, the present invention is not limited thereto. For example, when a branched chain polyglycerol is used as the raw material, the polyglycerol is ketalized or acetalized at positions 1 and 3; thus this type of compound can also be used.

When the addition of the alkylene oxide is carried out to the compound of formula (4) in the presence of an alkaline catalyst, a reaction is normally conducted at 40 to 180 ˚C in a high pressure reactor such as an autoclave. On this occasion, oxides, hydroxides, alcoholates, etc. of alkali metals and alkaline earth metals may be used as a catalyst. Specific examples thereof include sodium hydroxide, potassium hydroxide, calcium hydroxide, calcium oxide, and sodium methoxide. The amount of catalyst is not limited in particular, and 0.01 to 5.0 mass % with respect to the weight after the addition reaction is generally used.

After the alkylene oxide addition reaction, alkyl (alkenyl) etherification of the oxyalkylene terminal can be carried out, if necessary, by reacting with alkyl (alkenyl) halide in the presence of an alkaline catalyst. Examples of alkyl (alkenyl) halides include methyl chloride, ethyl chloride, propyl chloride, butyl chloride, vinyl chloride, allyl chloride, methyl bromide, ethyl bromide, methyl iodide, and ethyl iodide. As the catalyst, oxides, hydroxides, or alcoholates of alkali metals and alkaline earth metals can be used. Specific examples thereof include sodium hydroxide, potassium hydroxide, calcium hydroxide, calcium oxide, and sodium methoxide. The amount of loaded alkyl (alkenyl) halide is 100 to 400 mol % with respect to the reacting hydroxyl groups. The amount of the alkaline catalyst is 100 to 500 mol % with respect to the reacting hydroxyl groups. The reaction temperature is generally 60 to 160 ˚C.

When the succeeding deketalization or deacetalization of the oxyalkylenated compound of formula (4) is carried out, it is necessary to conduct neutralization with an acid, alkali adsorption, filtration, etc.. For example, neutralizing agents including mineral acids such as hydrochloric acid, sulfuric acid, phosphoric acid, acetic acid, and carbonic acid and organic acids such as citric acid, succinic acid, and tartaric acid; adsorbents such as Kyowado 600 and Kyowado 700 of Kyowa Chemical Industry Co., Ltd. and Tomix AD-300 of Tomita Pharmaceutical Co., Ltd.; and zeolites can be used.

Deketalization or deacetalization of the oxyalkylenated compound of formula (4) is carried out in the presence of an acid catalyst. Examples of acid catalysts include hydrochloric acid, sulfuric acid, phosphoric acid, p-toluenesulfonic acid, other solid acids, cation exchange resins, and acid clays. The amount of the used acid catalyst is 0.01 to 6.0 mass

% with respect to the oxyalkylenated compound of formula (4). The reaction temperature is generally 60 to 150 °C.

After the completion of deketalization or deacetalization, neutralization with an alkali, acid adsorption, filtration, etc. be conducted. For example, neutralizing agents such as sodium hydroxide, potassium hydroxide, sodium carbonate, and sodium acetate; adsorbents such as Kyowado 300 and Kyowado 1000 of Kyowa Chemical Industry Co., Ltd. and Tomix AD-500 of Tomita Pharmaceutical Co., Ltd.; and zeolites can be used.

As explained above, a series of processes are used in the preparation of the polyglycerol derivative used in the present invention. First, the hydroxyl groups at the terminals of polyglycerol are beforehand protected by diketalization of diacetalization. Second, in this state, the oxyalkylenation of hydroxyl groups are carried out. Finally the protecting groups are removed by deketalization or deacetalization. Thus, polyglycerol derivatives shown in formula (1), in which only the hydroxyl groups at non-terminal sections of polyglycerol are selectively oxyalkylenated, can be obtained.

By using the thus obtained polyglycerol derivative of the specific structure as an emulsifier, a water-in-oil type external skin preparation excellent in emulsion stability can be obtained. In the water-in-oil type external skin preparation of the present invention, the blending quantity of the polyglycerol derivative is not limited in particular, and can be suitably adjusted in accordance with the intended use. The blending quantity, however, is preferably 0.5 to 30 mass % of the total composition, and more preferably 1 to 10 mass %. If the blending quantity of the polyglycerol derivative is less than 0.5 mass %, the emulsion stability may be poor. On the other hand, if the blending quantity is more than 30 mass %, the emulsion stability is poor and undesirable stickiness is caused, leading to a poor feeling in use.

By blending the thus obtained polyglycerol derivative of the specific structure, an oil-in-water type external skin preparation excellent in feeling in use and emulsion stability can be obtained. In the oil-in-water type external skin preparation of the present invention, the blending quantity of the polyglycerol derivatives is not limited in particular, and can be suitably adjusted in accordance with the intended use. The blending quantity, however, is preferably 0.2 to 7.0 mass % of the total composition, and more preferably 0.5 to 3.0 mass %. In the oil-in-water type external skin preparation containing the below-described inulin derivative and/or block-type alkylene oxide derivative and hydrophobized powder, the blending quantity of the polyglycerol derivative is preferably 0.2 to 5.0 mass % of the total composition, and more preferably 0.5 to 2.0 mass %. If the blending quantity of the polyglycerol derivative is less than 0.2 mass %, the emulsion stability may be poor. On the other hand, if the blending quantity is more than the above-described range, undesirable phase inversion to a water-in-oil type may take place.

In the following, as emulsified external skin preparations of the present invention, water-in-oil type external skin preparations will be initially explained, and then oil-in-water type external skin preparations will be explained.

## I. Water-in-oil type external skin preparations

### Polar oil component

The water-in-oil type external skin preparation of the present invention is extremely excellent in emulsion stability, when a polar oil component is blended as an oil component, because of the blending of the above-described polyglycerol derivative of the specific structure. Thus, the water-in-oil type external skin preparation of the present invention is especially useful when a polar oil component is also contained. Polar oil components used in the present invention are not limited in particular; however, it is desirable that the IOB value is 0.05 to 0.80.

IOB value is an abbreviation for the inorganic/organic balance, which shows the ratio of the inorganic value and the organic value and indicates the degree of polarity of an organic compound. More specifically, the IOB value is expressed:

$$\text{IOB value} = \text{inorganic value}/\text{organic value}$$

The "inorganic value" and the "organic value" are set for various atoms or functional groups. For example, the "organic value" is 20 per carbon atom in a molecule, and the "inorganic value" is 100 per hydroxyl group in a molecule. The IOB value of an organic compound can be calculated by adding the "inorganic values" and "organic values" of all atoms and functional groups in the organic compound, (refer to "Fujita, Kagaku No Ryoiki, Vol. 11, No. 10, 719-725, 1957", for example).

Examples of polar oils used in the present invention include isopropyl myristate, cetyl octanoate, octyldodecyl myristate, isopropyl palmitate, butyl stearate, hexyl laurate, myristyl myristate, decyl oleate, hexyldecyl dimethyloctanoate, cetyl lactate, myristyl lactate, lanolin acetate, isocetyl stearate, isocetyl isostearate, cholesteryl 12-hydroxystearate, ethylene glycol di-2-ethylhexanoate, dipentaerythritol fatty acid ester, N-alkyl glycol monoisostearate, neopentylglycol dicaprate, diisostearyl malate, glyceryl di-2-heptylundecanoate, trimethylolpropane tri-2-ethylhexanoate, trimethylolpropane triisostearate, pentaerythritol tetra-2-ethylhexanoate, glyceryl tri-2-ethylhexanoate, trimethylolpropane triisostearate, cetyl 2-ethylhexanoate, 2-ethylhexyl palmitate, $C_{12-15}$ alkyl benzoate, cetearyl isononanoate, caprylic/capric triglyceride, butylene glycol dicaprylate/dicaprate, glycerol trimyristate, glycerol tri-2-heptylundecanoate, castor oil fatty acid methyl ester, oleyl oleate, cetearyl alcohol, acetoglyceride, 2-heptylundecyl palmitate, diisobutyl adipate, 2-octyldodecyl N-lauroyl-L-glutamate, di-2-heptylundecyl adipate, ethyl laurate, di-2-ethylhexyl sebacate, 2-hexyldecyl myristate, 2-hexyldecyl palmitate, 2-hexyldecyl adipate, diisopropyl sebacate, di-2-ethylhexyl succinate, ethyl acetate, butyl acetate, amyl acetate, triethyl citrate, 2-ethylhexyl p-methoxycinnamate, tripropylene glycol dipivalate, and 2-ethylhexyl 2-cyano-3,3-diphenylacrylate

Among these polar oil components, it is especially desirable that the polar oil component is one or more selected from the group consisting of 2-ethylhexyl p-methoxycinnamate, 2-ethylhexyl 2-cyano-3,3-diphenylacrylate, tripropylene glycol dipivalate, cetyl octanoate, trimethylolpropane tri-2-ethylhexanoate, pentaerythritol tetra-2-ethylhexanoate, glyceryl tri-2-ethylhexanoate, $C_{12-15}$ alkyl benzoate, caprylic/capric triglyceride, propylene glycol dicaprylate/dicaprate, and di-2-ethylhexyl succinate.

In the water-in-oil type external skin preparation of the present invention, one or more selected from the above-described polar oil components may be used. The blending quantity of the polar oil component is preferably 0.1 to 90.0 mass % of the composition, and more preferably 1.0 to 70.0 mass %. If the blending quantity of the polar oil component is too small, stickiness may be caused, and if it is too much, the emulsion stability may be poor.

Silicone oil

The water-in-oil type external skin preparation of the present invention is extremely excellent in emulsion stability, when a silicone oil is blended as an oil component, because of the blending of the above-described polyglycerol derivative of the specific structure. Thus, the water-in-oil type external skin preparation of the present invention is especially useful when a silicone oil is also contained.

Examples of silicone oils include linear polysiloxanes such as dimethylpolysiloxane, methylphenylpolysiloxane, and methylhydrogenpolysiloxane; and cyclic polysiloxanes such as decamethylpolysiloxane, dodecamethylpolysiloxane, and tetramethyltetrahydrogenpolysiloxane.

In the water-in-oil type external skin preparation of the present invention, one or more selected from the above-described silicone oils may be used. The blending quantity of the silicone oil is preferably 0.1 to 90 mass % of the composition, and more preferably 1.0 to 70.0 mass %. If the blending quantity of the silicone oil is too small, stickiness may be caused, and if it is too much, the emulsion stability may be poor.

Solid UV absorber

In the water-in-oil type external skin preparation of the present invention, an UV absorber that is solid at ordinary temperature may preferably be blended in addition to the above-described essential components. Examples of UV absorbers that are solid at ordinary temperature and used in the present invention include 2,4-bis-[[4-( 2-ethylhexyloxy)-2-hydroxy]-phenyl]-6-(4-methoxyphenyl)-1,3,5-triazine (product name: Tinosorb S (Ciba)), 2,4,6-trianilino-(p-carbo-2'-ethylhexyl-1'-oxy)-1,3,5-triazine (product name: Uvinul T 150 (BASF)), and 1-[4-(1,1-dimethylethyl) phenyl]-3-(4-methoxyphenyl)-1,3-propanedione (product name: Palsol 1789 (Roche)).

In the water-in-oil type external skin preparation of the present invention, one or more selected from the above-described UV absorbers that are solid at ordinary temperature may be used. The blending quantity of the above-described UV absorber that is solid at ordinary temperature is preferably 0.05 to 30 mass % of the composition, and more preferably 0.1 to 20.0 mass %. If the blending quantity of the UV absorber that is solid at ordinary temperature is too small, a sufficient UV protective effect may not be achieved, and if it is too much, the UV absorber may separate out with time.

UV scatterer

In the water-in-oil type external skin preparation of the present invention, an UV scatterer may preferably be blended in addition to the above-described essential components. Examples of UV scatterers used in the present invention include inorganic powders such as titanium oxide and zinc oxide; and surface-coated inorganic powders, in which the surface of the inorganic powders is coated with fatty acid soaps such as aluminum stearate and zinc palmitate; fatty acids such as stearic acid, myristic acid, and palmitic acid; or fatty acid esters such as dextrin palmitate.

In the water-in-oil type external skin preparation of the present invention, one or more selected from the above-described UV scatterers may be used. The blending quantity of the UV scatterer is preferably 0.05 to 30.0 mass % of the composition, and more preferably 0.1 to 20.0 mass %. If the blending quantity of the UV scatterer is too small, a sufficient UV protective effect may not be achieved, and if it is too much, an emulsion may not be obtained.

In the water-in-oil type external skin preparation of the present invention, powder with an UV shielding effect may preferably be blended. Examples of these powders include inorganic powders of talc, kaolin, mica, sericite, muscovite, phlogopite, synthetic mica, lepidolite, biotite, lithia mica, vermiculite, magnesium carbonate, calcium carbonate, aluminum silicate, barium silicate, calcium silicate, magnesium silicate, strontium silicate, metal tungstates, magnesium, silica, zeolite, barium sulfate, calcined calcium sulfate (calcined gypsum), calcium phosphate, fluoroapatite, hydroxyapatite, ceramic powder, metallic soap (zinc myristate, calcium palmitate, aluminum stearate), boron nitride, titanium oxide, zinc oxide, etc.; organic powders of polyamide resins (Nylon), polyethylene, polymethyl methacrylate, polystyrene, styrene-acrylic acid copolymer resin, benzoguanamine resin, polytetrafluoroethylene, cellulose, etc.; inorganic red powders of red iron oxide and iron titanate, etc.; inorganic brown powders of y-iron oxide etc.; inorganic yellow powders of yellow iron oxide, ochre, etc.; inorganic black powders of black iron oxide, carbon black, low-order titanium oxide, etc.; inorganic violet powders of manganese violet, cobalt violet, etc.; inorganic green powders of chromium oxide, chromium hydroxide, cobalt titanate, etc.; inorganic blue powders of ultramarine, Prussian blue etc.; pearly powders of titanium oxide coated mica, titanium oxide coated bismuth oxychloride, titanium oxide coated talc, colored titanium oxide coated mica, bismuth oxychloride, fish scale flake, etc.; and metal powders such as aluminum powder and copper powder. The particle size of these powders can suitably be selected as necessary. In addition, they can be used in a form of hydrophobized powder, if necessary, by hydrophobizing them with silicone or fatty acids, for example.

The oil phase component blended in the water-in-oil type external skin preparations of the present invention is not limited to the above-described polar oils and silicone oils, and other oil components generally used in cosmetics can be blended. Other oil components can be nonpolar oils such as liquid hydrocarbons, semi-solid (grease-like) hydrocarbons, and solid hydrocarbons. Examples thereof include liquid paraffin, squalane, isoparaffin, ozokerite, pristane, ceresin, petrolatum, microcrystalline wax, and paraffin wax. The total amount of the oil components contained in the water-in-oil type external skin preparation of the present invention is not limited in particular. It is preferably about 20 to 95 mass % of the total composition, and more preferably 30 to 80 mass %. If the total amount of the oil components is less than 20 mass %, it is difficult to achieve a good feeling in use as an external preparation. On the other hand, if it exceeds 95 mass %, the emulsion stability often becomes poor with time.

The water phase component is also not limited in particular. In addition to water, it is possible to blend one or more of mixtures with, for example, monohydric alcohols such ethanol, propanol, isopropanol, butanol, and benzyl alcohol; glycols such ethylene glycol, propylene glycol, 1,3-butylene glycol, 1,4-butylene glycol, hexylene glycol, and dipropylene glycol; glycerols such as glycerol, diglycerol, triglycerol, and higher polyglycerol; polyalkylene glycols such as polyethylene glycol and polypropylene glycol; dialkyl ethers such as PEG (14) PPG (7) dimethyl ether and PEG (36) PPG (41) dimethyl ether; or polyhydric alcohols, which contain two or more hydroxyl groups in a molecule, such as glucose, maltose, maltitol, sucrose, and sorbitol.

In the water-in-oil type external skin preparations of the present invention, various components normally used in external preparations can be blended, as necessary, so far as the effect of the present invention is not undermined; their examples include powder components, liquid fats, solid fats, waxes, hydrocarbons, higher fatty acids, higher alcohols, anionic surfactants, cationic surfactants, amphoteric surfactants, nonionic surfactants, moisturizers, water-soluble polymers, thickeners, metal ion sequestering agents, lower alcohols, polyhydric alcohols, saccharides, amino acids, organic amines, polymer emulsions, coloring matters, pH adjusters, skin nutrients, vitamins, preservatives, antioxidants, antioxidant aids, perfumes, and water, and normal preparation methods can be used in accordance with the desired product forms.

The usage of the water-in-oil type external skin preparation of the present invention is not limited in particular. For example, they can be used for various products such as lotion, milky lotion, cream, foundation, lipstick, cleansing foam, shampoo, hair rinse, lip cream, hair spray, mousse, sunscreen or suntan cream, eye liner, mascara, hair care or nail care, cream, and body makeup preparations. Among these, the preparation can be preferably used as a sunscreen.

## II. Oil-in-water type external skin preparations

### Hydrophobized powder

The oil-in-water type external skin preparation of the present invention is extremely excellent in dispersion stability, when hydrophobized powder is blended, because of the blending of the above-described polyglycerol derivative of the specific structure. Thus, the oil-in-water type external skin preparation of the present invention is especially useful when hydrophobized powder is also contained.

Hydrophobized powder used in the present invention is not limited in particular so far as the surface of powder is hydrophobized. For example, the surface of inorganic powder can be hydrophobized by a wet method with the use of solvent, gas phase method, or a mechanochemical method with silicones such as methylhydrogenpolysiloxane or dimethylpolysiloxane; dextrin fatty acid esters; higher fatty acids; higher alcohols; fatty acid esters; metallic soap; alkyl phosphate ethers; fluorine compounds; or hydrocarbons such as squalane or paraffins. The average particle size of hydrophobized powder needs to be smaller than the size of emulsified particles, which is the oil phase in the present invention. Especially when the powder is used as an UV scatterer, the average particle size after crushing with a wet

disperser should preferably be 100 nm or less. Examples of inorganic powders, which are to be hydrophobized, include titanium oxide, zinc oxide, talc, mica, sericite, kaolin, titanated mica, black iron oxide, yellow iron oxide, red iron oxide, ultramarine, Prussian blue, chromium oxide, and chromium hydroxide. Among these hydrophobized powders, hydrophobized particulate titanium dioxide and/or hydrophobized particulate zinc oxide are preferably used.

The blending quantity of hydrophobized powder in the oil-in-water type external skin preparation of the present invention is preferably 0.1 to 20 mass % of the total composition. If the blending quantity is less than 0.1 mass %, the blending effect is not satisfactory, and if it exceeds 20 mass %, the emulsion stability may become poor.

When hydrophobized powder is blended in the oil-in-water type external skin preparation of the present invention, hydrophobized powder and the above-described polyglycerol derivative are blended beforehand in the oil component, which constitutes the oil phase. Powder dispersion liquid is obtained by pulverizing the powder with a wet disperser with high crushing power such as a bead mill. Subsequently, the obtained powder dispersion liquid is mixed and emulsified with a homomixer, with the water phase containing emulsifiers (for example, the below-described inulin derivative and block-type alkylene oxide derivative). On this occasion, if powder particles with the size larger than that of the formed emulsified particles are present, part of the powder comes out of the oil phase during a homomixer treatment, resulting in the formation of aggregates. Therefore, it is necessary to allow the average particle size of the powder to be smaller than that of the oil phase. If a bead mill is used, it is possible to allow the particle size of pulverized powder to be sufficiently small and obtain pulverized powder sufficiently smaller than the size of the emulsified particles by increasing the number of passes of the dispersion liquid through the mill.

Inulin derivatives

The inulin derivative used in the oil-in-water type external skin preparation of the present invention is represented by the following formula (2-a):

$$A\text{-}(O\text{-}CO\text{-}NH\text{-}R^1)s \qquad (2\text{-}a)$$

wherein A is a fructose residue of inulin;

(O-CO-NH-$R^1$) represents a N-alkylaminocarbonyloxy group substituting a hydroxyl group of the fructose;
$R^1$ is a hydrocarbon group having 3 to 22 carbon atoms; and
s is the substitution degree of the N-alkylaminocarbonyloxy group per fructose residue, and s takes 0.10 to 2.0.

In the inulin derivative represented by formula (2), A is a fructose residue of inulin. Inulin is a polysaccharide, in which D-fructose residues are connected through $\beta 2 \rightarrow 1$ linkage, and the D-fructose at the terminal is connected to D-glucose through a sucrose-type linkage. As the inulin, a hydrolysis product of inulin (oligofructose) having the average polymerization degree of D-fructose units of about 3 to 70 may be used. In the inulin derivative of the present invention, the molecular weight of the inulin is not limited in particular.

N-alkylaminocarbonyloxy group is represented by (O-CO-NH-$R^1$), which substitute a hydroxyl group of a fructose. For example, a fructose hydroxyl group is substituted with N-alkylaminocarbonyloxy group represented by -(O-CO-NH-$R^1$) by the reaction of an alkylisocyanate to a fructose hydroxyl group of inulin. $R^1$ is a hydrocarbon group having 3 to 22 carbon atoms, and it can be either linear or branched and either saturated or unsaturated. Examples of hydrocarbon groups include saturated hydrocarbon groups such as an n-propyl group, an isopropyl group, an n-butyl group, a t-butyl group, a hexyl group, a decyl group, a dodecyl group, a tetradecyl group, a hexadecyl group, and an octadecyl group; unsaturated hydrocarbon groups such as a hexenyl group, an octenyl group, a decenyl group, a dodecenyl group, a tetradecenyl group, a hexadecenyl group, and an octadecenyl group; and a mixture thereof, and a saturated hydrocarbon group is preferable.

The degree of substitution with N-alkylaminocarbonyloxy group per fructose residue is represented by s, and s takes 0.10 to 2.0. The number of hydroxyl groups substitutable with an N-alkylaminocarbonyloxy group is three per fructose residue, and s is expressed by the average number of substitution degree per fructose residue in an inulin derivative.

As the above-described inulin derivative, a commercial product can be used. As a commercial inulin derivative, for example, INUTEC SP (product of ORAFTI) etc. can be used in the present invention.

In the oil-in-water type external skin preparation of the present invention, the blending quantity of the above-described inulin derivative is not limited in particular, and can be suitably adjusted in accordance with the intended use: however, it is preferably 0.1 to 6 mass % of the total composition, and more preferably 0.5 to 4 mass %. If the blending quantity is less than 0.1 mass %, the emulsification may not be satisfactory. If the blending quantity exceeds 6 mass %, a sticky feeling may be caused after application.

The oil-in-water type external skin preparation of the present invention is extremely excellent in feeling in use,

powder dispersion stability, and emulsion stability, when hydrophobized powder is blended, because of the blending of both the above-described polyglycerol derivative of a specific structure and the above-described inulin derivative of a specific structure.

Among these hydrophobized powders, especially when both hydrophobized particulate titanium dioxide and hydrophobized particulate zinc oxide are blended, significant aggregation and coalescence of emulsified particles are known to occur. In the oil-in-water type external skin preparation of the present invention, it is possible to significantly improve the powder dispersion stability and emulsion stability by blending both the above-described polyglycerol derivative of a specific structure and the above-described inulin derivative of a specific structure. Thus, the oil-in-water type external skin preparation of the present invention is especially useful when hydrophobized particulate titanium dioxide and hydrophobized particulate zinc oxide are contained as the hydrophobized powder.

Block-type alkylene oxide derivative

The block-type alkylene oxide derivative used in the oil-in-water type external skin preparations of the present invention is represented by formula (2-b):

$$R^1O\text{-}(AO)_m(EO)_n\text{-}R^2 \qquad (2\text{-}b)$$

In the alkylene oxide derivative represented by the above-described formula (2-b), AO is an oxyalkylene group having 3 to 4 carbon atoms. The specific examples of oxyalkylene groups include oxypropylene group, oxybutylene group, oxyisobutylene group, oxytrimethylene group, and oxytetramethylene group; and an oxypropylene group or an oxybutylene group is preferable.

The value m is the average addition mole number of the oxyalkylene group having 3 to 4 carbon atoms, and $1 \leqq m \leqq 70$, preferably $2 \leqq m \leqq 50$. The value n is the average addition mole number of the oxyethylene group, and $1 \leqq n \leqq 70$, preferably $5 \leqq n \leqq 55$. If the number of oxyalkylene groups having 3 to 4 carbon atoms or the number of the oxyethylene group is zero, a moist feeling decreases. If it exceeds 70, a sticky feeling is produced.

It is preferable that the percentage of the oxyethylene group with respect to the sum of oxyalkylene groups having 3 to 4 carbon atoms and oxyethylene group is 20 to 80 mass %. If the percentage of the oxyethylene group is less than 20 mass %, a phase inversion to the water-in-oil type may take place. If it is more than 80 mass %, the emulsion stability tends to be poor.

The oxyethylene group and oxyalkylene group having 3 to 4 carbon atoms should be added in a block-type. However, the addition order of ethylene oxide and alkylene oxide having 3 to 4 carbon atoms is not specified. The block-type includes not only two-stepwise block but also three- or more-stepwise block.

$R^1$ and $R^2$ are hydrocarbon groups having 1 to 4 carbon atoms or hydrogen atoms. Examples of hydrocarbon groups include methyl group, ethyl group, n-propyl group, isopropyl group, n-butyl group, see-butyl group, and tert-butyl group; and methyl group or ethyl group is preferable. If the hydrocarbon group has 5 or more carbon atoms, the hydrophilicity decreases and skin irritation tends to occur. $R^1$ and $R^2$ may be the same or different.

$R^1$ and $R^2$ may be the same. Alternatively, in $R^1$ and $R^2$, hydrocarbon groups having 1 to 4 carbon atom and hydrogen atoms may be present together, or different hydrocarbon groups having 1 to 4 carbon atoms may be present. However, the ratio of hydrocarbon groups and hydrogen atoms in $R^1$ and $R^2$, namely, the ratio of the number of hydrogen atom (Y) with respect to the number of hydrocarbon group (X), Y/X, should be 0.15 or less, and more preferably 0.06 or less. If the ratio Y/X exceeds 0.15, a sticky feeling is generated.

The molecular weight of the block-type alkylene oxide derivative is preferably 1000 or higher, and more preferably 3000 or higher. If the molecular weight is less than 1000, the emulsion stability is low. Although the upper limit of the molecular weight cannot be specified, a sticky feeling after application tends to occur with an increase of the molecular weight.

Specific examples of block-type alkylene oxide derivatives used in the present invention include POE (14) POP (7) dimethyl ether, POE (17) POP (4) dimethyl ether, POE (10) POP (10) dimethyl ether, POE (7) POP (12) dimethyl ether, POE (15) POP (5) dimethyl ether, POE (25) POP (25) dimethyl ether, POE (27) POP (14) dimethyl ether, POE (55) POP (28) dimethyl ether, POE (22) POP (40) dimethyl ether, POE (35) POP (40) dimethyl ether, POE (50) POP (40) dimethyl ether, POE (36) POP (41) dimethyl ether, POE (55) POP (30) dimethyl ether, POE (30) POP (34) dimethyl ether, POE (25) POP (30) dimethyl ether, POE (14) POB (7) dimethyl ether, POE (10) POP (10) diethyl ether, POE (10) POP (10) dipropyl ether, and POE (10) POP (10) dibutyl ether.

The abbreviations POE, POP, and POB, which are used above, are for polyoxyethylene, polyoxypropylene, and polyoxybutylene, respectively; hereinafter these abbreviations may be used.

The block-type alkylene oxide derivative used in the present invention can be prepared by a known method. For example, after addition polymerization of ethylene oxide and an alkylene oxide having 3 to 4 carbon atoms with a compound with a hydroxyl group, etherification with an alkyl halide is carried out in the presence of an alkaline catalyst,

to obtain the product.

In the oil-in-water type external skin preparation of the present invention, the blending quantity of the above-described block-type alkylene oxide derivative is not limited in particular, and can be suitably adjusted in accordance with the intended use; however, it is preferably 0.3 to 6 mass % of the total composition, and more preferably 0.5 to 4 mass %. If the blending quantity is less than 0.3 mass %, the emulsification may not be satisfactory. If it exceeds 6 mass %, a sticky feeling may occur after application.

The oil-in-water type external skin preparation of the present invention is extremely excellent in feeling in use, powder dispersion stability, and emulsion stability, when hydrophobized powder is blended, because of the blending of both the above-described polyglycerol derivative of a specific structure and the above-described block-type alkylene oxide derivative of a specific structure.

Among these hydrophobized powders, especially when both hydrophobized particulate titanium dioxide and hydrophobized particulate zinc oxide are blended, significant aggregation and coalescence of emulsified particles are known to occur. In the oil-in-water type external skin preparation of the present invention, it is possible to significantly improve the powder dispersion stability and emulsion stability by blending both the above-described polyglycerol derivative of a specific structure and the above-described block-type alkylene oxide derivative of a specific structure. Thus, the oil-in-water type external skin preparation of the present invention is especially useful when hydrophobized particulate titanium dioxide and hydrophobized particulate zinc oxide are contained as the hydrophobized powder.

Salt-tolerant thickener such as succinoglycan

In the oil-in-water type external skin preparation of the present invention, the long-term stability against sedimentation and creaming of emulsified oil drops and the long-term stability against powder aggregation can be improved by blending a salt-tolerant thickener, specifically succinoglycan, xanthan gum, or acrylamide. When a common thickener such as polyacrylic acid is used, the thickener is affected by salts gradually leach out, with time, from the inorganic powder fine particles into the water phase. As a result, the viscosity may be decreased. In contrast, when an excellent salt-tolerant thickener such as succinoglycan is used, there is no effect from the leached-out salts from inorganic powder. Thus, the powder aggregation and the sedimentation of emulsified particles can be avoided for a long period. The blending quantity of such a salt-tolerant thickener is preferably 0.1 to 1 mass % of the total composition. If the blending quantity is less than 0.1 mass %, the blending effect is not satisfactory. If it exceeds 1 mass %, the feeling in use may be poor due to lumps, etc..

As the salt-tolerant thickener, the use of succinoglycan is especially desirable because it has the large retaining power under a temperature change and the high yield value. In addition, the feeling in use is excellent in that it is not powdery and provides a fresh feeling. Succinoglycan is a kind of polysaccharide and is derived from microbes. Specifically, succinoglycan means microbe-derived polysaccharides, which contain, in addition to the sugar units derived from galactose and glucose, a succinic acid unit and a pyruvic acid unit, and optionally contain an acetic acid unit or the units derived from salts of these acids.

More specifically, succinoglycan is a water-soluble polymer represented by the below-described structural formula, in which the average molecular weight is about 6,000,000 or less, and one galactose unit, seven glucose units, 0.8 succinic acid units, one pyruvic acid unit, and an optional acetic acid unit are contained.

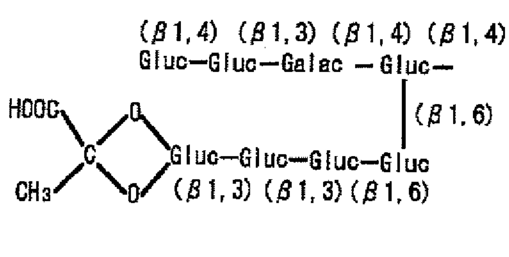

In the formula, Gluc represents a glucose unit and Galac represents a galactose unit. The representation in the parentheses indicates bonding patterns between the sugar units. For example, (β1,4) indicates β1-4 linkage.

Examples of microbes, which are sources of this succinoglycan, include bacteria that belong to the Pseudomonas, Rhizobium, Alcaligenes, or Agrobacterium. Among these bacteria, a bacterium that belongs to the Agrobacterium, Agrobacterium tumefaciens I-736 (deposited on March 1, 1988 to the Collection Nationale de Cultures de Microorganismes (CNCM) following the Budapest Treaty, publicly-obtainable using accession No. I-736.), is particularly preferable as the source of succinoglycan.

Succinoglycan can be produced by culturing these microbes in a medium. More specifically, succinoglycan is

generally produced by culturing the above-descried microbes in the medium containing carbon sources such as glucose, sucrose, and hydrolysis products of starch; organic nitrogen sources such as casein, caseinate, vegetable powder, yeast extract, and corn steep liquor (CSL); inorganic salts such as metal sulfates, metal phosphates, and metal carbonates; and optional trace elements.

Into the oil-in-water type external skin preparations of the present invention, the thus produced succinoglycan can be obviously blended as it is, and the degradation products by acid decomposition, alkaline decomposition, enzymatic decomposition, and ultrasonic treatment can also be blended as necessary. When succinoglycan is used as a thickener, powder lumps may occasionally be generated upon application of the composition on the skin. In order to remedy this problem, it is particularly preferable to use dynamite glycerol as a moisturizer together. Thus, powder lumps disappear and the feeling in use can be improved.

Emulsifying aids such as carboxymethylcellulose

In order to improve temperature stability and powder dispersion stability of the oil-in-water type external skin preparations of the present invention, it is desirable to blend one or more selected from the group consisting of carboxymethylcellulose, hydroxyethyl cellulose, hydroxymethyl cellulose, and gelatin in a quantity of 0.1 to 1.0 mass % as an emulsifying aid. If the blending quantity is less than 0.1 mass %, the blending effect is not satisfactory. If it exceeds 1.0 mass %, the feeling in use tends to be poor.

Oil components

The oil component blended in the oil-in-water type external skin preparation of the present invention is not limited in particular. For example, silicone oils or polar oils can be preferably used. Examples of silicone oils include linear or cyclic polysiloxanes such as dimethylpolysiloxane, methylphenylpolysiloxane, methylhydrogenpolysiloxane, decamethylpolysiloxane, dodecamethylpolysiloxane, tetramethyltetrahydrogenpolysiloxane, cyclotetradimethylsiloxane, and cyclopentadimethylsiloxane.

Examples of polar oils include synthetic ester oils, natural ester oils, and specific UV absorbers. Examples of synthetic ester oils include isopropyl myristate, cetyl octanoate, octyldodecyl myristate, isopropyl palmitate, butyl stearate, hexyl laurate, myristyl myristate, decyl oleate, hexyldecyl dimethyloctanoate, cetyl lactate, myristyl lactate, lanolin acetate, isocetyl stearate, isocetyl isostearate, cholesteryl 12-hydroxystearate, ethylene glycol di-2-ethylhexanoate, dipentaerythritol fatty acid ester, N-alkyl glycol monoisostearate, neopentylglycol dicaprate, diisostearyl malate, glyceryl di-2-heptylundecanoate, trimethylolpropane tri-2-ethylhexanoate, trimethylolpropane triisostearate, pentaerythritol tetra-2-ethylhexanoate, glycerol tri-2-ethylhexanoate, trimethylolpropane triisostearate, cetyl 2-ethylhexanoate, 2-ethylhexyl palmitate, glycerol trimyristate, glyceride tri-2-heptylundecanoate, castor oil fatty acid methyl ester, oleyl oleate, cetearyl alcohol, acetoglyceride, 2-heptylundecyl palmitate, diisobutyl adipate, 2-octyldodecyl N-lauroyl-L-glutamate, di-2-heptylundecyl adipate, ethyl laurate, di-2-ethylhexyl sebacate, 2-hexyldecyl myristate, 2-hexyldecyl palmitate, 2-hexyldecyl adipate, diisopropyl sebacate, 2-ethylhexyl succinate, ethyl acetate, butyl acetate, amyl acetate, and triethyl citrate. Examples of natural ester oils include avocado oil, camellia oil, turtle oil, macadamia nut oil, corn oil, mink oil, olive oil, rapeseed oil, egg-yolk oil, sesame oil, persic oil, wheat germ oil, sasanqua oil, castor oil, linseed oil, safflower oil, cottonseed oil, perilla oil, soybean oil, peanut oil, tea seed oil, kaya oil, rice bran oil, paulownia oil, jojoba oil, germ oil, triglycerol, glyceryl trioctanoate, glyceryl triisopalmitate. Examples of UV absorbers, which are polar oils, include cinnamate series UV absorbers such as octyl cinnamate, ethyl 4-isopropyl cinnamate, ethyl 2,4-diisopropyl cinnamate, methyl 2,4-diisopropyl cinnamate, propyl p-methoxycinnamate, isopropyl p-methoxycinnamate, isoamyl p-methoxycinnamate, octyl methoxycinnamate, 2-ethoxyethyl p-methoxycinnamate, cyclohexyl p-methoxycinnamate, ethyl a-cyano-6-phenylcinnamate, and 2-ethylhexyl $\alpha$-cyano-$\beta$-phenylcinnamate

In addition to the above-described polar oils and silicone oils, other oil components that are used in common external preparations can be used as the oil components blended in the oil-in-water type external skin preparation of the present invention. For example, nonpolar oils such as liquid hydrocarbons, semi-solid (grease-like) hydrocarbons, and solid hydrocarbons can be used. Examples thereof include liquid paraffin, squalane, isoparaffin, ozokerite, pristane, ceresin, petrolatum, microcrystalline wax, and paraffin wax. The content of total oil components in the oil-in-water type external skin preparation of the present invention is not limited in particular; it is about 5 to 60 mass % of the total composition, and it is preferably 10 to 35 mass %. If the content of total oil components is less than about 5 mass %, it is difficult to achieve a good feeling in use as an external preparation. On the other hand, if it exceeds 60 mass %, the long-term emulsion stability may become poor.

Emulsifiers

As the emulsifier used in the oil-in-water type external skin preparation of the present invention, the above-described inulin derivatives or block-type alkylene oxide derivatives can be preferably used. Other emulsifiers, however, can be used so far as the effect of the present invention is not undermined. Such emulsifiers are not limited in particular; however, hydrophilic surfactants are desirable because their solubility in the oil phase is low and the temperature stability is good. In particular, the emulsifier that consists of one or more surfactants with the total HLB of 10 or more is desirable. Specifically, one or more selected from the group consisting of glycerol or polyglycerol fatty acid esters, propylene glycol fatty acid esters, POE sorbitan fatty acid esters, POE sorbitol fatty acid esters, POE glycerol fatty acid esters, POE fatty acid esters, POE alkyl ethers, POE alkylphenyl ethers, POE/POP alkyl ethers, POE castor oil or hydrogenated castor oil derivatives, POE beeswax/lanolin derivatives, alkanolamides, POE propylene glycol fatty acid esters, POE alkylamines, and POE fatty acid amides, can be blended. The blending quantity of the emulsifier is preferably 0.1 to 6 mass % of the total composition, and more preferably 0.5 to 5 mass %.

In the oil-in-water type external skin preparation of the present invention, various components normally used in external preparations can be blended so far as the effect of the present invention is not undermined. Examples thereof include moisturizers, UV absorbers, pH adjusters, neutralizing agents, antioxidants, preservatives, antibacterial agents, drugs, extracts, perfumes, and coloring matters. Examples of moisturizers include polyhydric alcohols such as glycerol, diethylene glycol, butylene glycol, and polyethylene glycol; amino acids; nucleic acids; proteins such as collagen and elastin; and mucopolysaccharides such as hyaluronic acid and chondroitin sulfate.

Examples of UV absorbers include benzoic acid UV absorbers such as p-aminobenzoic acid; anthranilic acid UV absorbers such as methyl anthranilate; salicylic acid UV absorbers such as octyl salicylate, phenyl salicylate, and homomethyl salicylate; cinnamic acid UV absorbers such as isopropyl p-methoxycinnamate, octyl p-methoxycinnamate, 2-ethylhexyl p-methoxycinnamate, glyceryl mono-2-ethylhexanoate di-p-methoxycinnamate, [4-bis(trimethylsiloxy) methylsilyl-3-methylbutyl]-3,4,5,-trimethoxy cinnamic acid ester; benzophenone UV absorbers such as 2,4-dihydroxybenzophenone, 2-hydroxy-4-methoxybenzophenone, 2-hydroxy-4-methoxybenzophenone-5-sulfonic acid, sodium 2-hydroxy-4-methoxybenzophenone-5-sulfonate; urocanic acid; ethyl urocanate; 2-phenyl-5-methylbenzoxazole, 2-(2'-hydroxy-5'-methylphenyl)benzotriazole; 4-tert-butyl-4'-methoxybenzoylmethane, bis(resorcinyl)triazine; and 2,4-bis[[4-(2-ethylhexyloxy)-2-hydroxy]-phenyl]-6-(4-methoxyphenyl)-1,3,5-triazine.

Examples of pH adjusters include lactic acid, citric acid, glycolic acid, succinic acid, tartaric acid, DL-malic acid, potassium carbonate, sodium hydrogencarbonate, and ammonium hydrogencarbonate. Examples of antioxidants include ascorbic acid, α-tocopherol, dibutylhydroxytoluene, and butylhydroxyanisole. Examples of preservatives and antibacterial agents include paraoxybenzoic acid ester, phenoxyethanol, benzoic acid, salicylic acid, carbolic acid, sorbic acid, p-chloro-m-cresol, hexachlorophene, benzalkonium chloride, chlorhexidine chloride, trichlorocarbanilide, and photosensitizers.

The application of the oil-in-water type external skin preparations of the present invention is not limited in particular. They can be applied for various products such as lotion, milky lotion, cream, foundation, lipstick, cleansing foam, shampoo, hair rinse, lip cream, hair spray, mousse, sunscreen or suntan cream, eye liner, mascara, hair care or nail care, cream, and body makeup preparations. Among these, the application to sunscreen is particularly desirable.

## EXAMPLES

The present invention will hereinafter be described in further detail with reference to examples. However, the present invention is not limited to these examples.
At first, a producing method of polyglycerol derivatives used in the present invention will be described.

Synthesis Example 1 Polyoxybutylene(25 mol)methyl triglyceryl ether

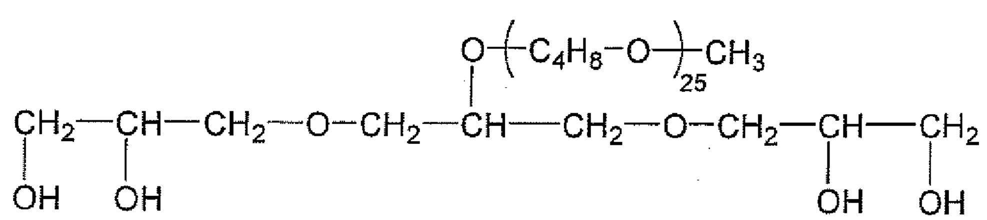

(1) Ketalization

Into a four-neck flask, 240 g of triglycerol (product of SOLVAY, "Triglycerin> 80%", purity: 83%), 364g of 2,2-dimethoxypropane, and 1.5 mg ofp-toluenesulflonic acid were loaded, the inner atmosphere of the reaction system was replaced with nitrogen gas, and the reaction was carried out for 3 hours at 50 ˚C. After the reaction, unreacted volatile components were distilled away by heating under a nitrogen stream, and acetic acid was added to adjust the pH to 7; thus a diketalized triglycerol was obtained. The purity of triglycerol was determined under the above-described GC analysis conditions. When the IR results for the raw material triglycerol and the product were compared, it was found that a peak in the vicinity of 3500 $cm^{-1}$ due to hydroxyl groups had become small on the product IR. Alternatively, peaks appeared in the vicinities of 2960 $cm^{-1}$, 2870 $cm^{-1}$, 1460 $cm^{-1}$, and 1380 $cm^{-1}$; thus it was confirmed that the desired product was obtained.

(2) Oxybutylenation

Into an autoclave, 320 g of the diketalized triglycerol and 12 g of potassium hydroxide were loaded, the air in the autoclave was replaced with dry nitrogen, and then the catalyst was completely dissolved with stirring at 140 ˚C. Subsequently, 1800 g of butylene oxide was dropwise added from a dropping apparatus, and the solution was stirred for 2 hours. Subsequently, 168.3 g of potassium hydroxide was loaded, the inner atmosphere of the system was replaced with dry nitrogen, 151.5 g of methyl chloride was introduced under pressure at 80 to 130 ˚C, and a reaction was carried out for 5 hours. Then the reaction product was removed from the autoclave, neutralized with hydrochloric acid to adjust the pH to 6 to 7, and then treated at 100 ˚C for 1 hour under reduced pressure to remove the contained water. The salt, which was produced after the treatment, was removed by filtration, to give an oxybutylenated diketalized triglycerol.

(3) Deketalization

Into a four-neck flask, 2134 g of the oxybutylenated diketalized triglycerol, 50 g of 36% hydrochloric acid, and 100 g of water were loaded, deketalization was carried out at 80 ˚C for 2 hours in a sealed condition. Subsequently, pH was adjusted to 6 to 7 with potassium hydroxide aqueous solution, and the mixture was treated at 100 ˚C for 1 hour under reduced pressure to remove the contained water. The salt, which was produced after the treatment, was removed by filtration, to give polyoxybutylene(25 mol)methyl triglyceryl ether.

The thus obtained product was analyzed by GPC, and the molecular weight of the main peak was found to be 1939. The analysis conditions were as follows.

Analytical instrument: SHODEX GPC SYSTEM-11 (product of Showa Denko K.K.)
Standard material: polyethylene glycol
Sample size: 10% $\times$ 100$\times$0.001 mL
Eluent: THF
Flow rate: 1.0 mL/min
Column: SHODEX KF804L (product of Showa Denko K.K.)
Column size: I.D. 8 mm $\times$ 30 cm $\times$ 3
Column temperature: 40 ˚C
Detector: RIx8

When the IR results for the oxybutylenated diketalized triglycerol and the product were compared, a hydroxyl group peak in the vicinity of 3500 $cm^{-1}$ has increased in the product. Thus, it was confirmed that the desired product has been obtained.

Synthesis Example 2 Polyoxvbutylene(25 mol)methyl triglyceryl ether

Among the procedures of the above-described Synthesis Example 1, (1) the ketalization procedure was changed as described below to obtain polyoxybutylene(25 mol)methyl triglyceryl ether. The conditions etc. are set according to those of Synthesis Example 1.

(1) Ketalization

Into a four-neck flask, 240 g of triglycerol (product of SOLVAY, "Triglycerin> 80%", purity: 83%), 290 g of acetone, and 4 mg of p-toluenesulfonic acid were loaded, the inner atmosphere of the reaction system was replaced with nitrogen gas, and the reaction was carried out at 70 ˚C for 8 hours. After the reaction, unreacted volatile components

were distilled away by heating under a nitrogen stream, and acetic acid was added to adjust the pH to 7; thus a diketalized triglycerol compound was obtained.

Synthesis Example 3 Polyoxybutylene(50 mol) triglyceryl ether

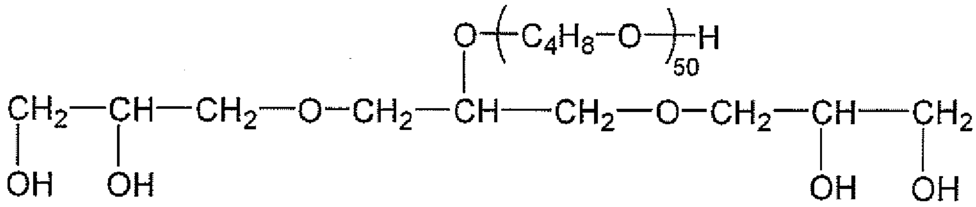

Among the procedures of the above-described Synthesis Example 1, (2) oxybutylenation and (3) deketalization were changed as described below to obtain polyoxybutylene(50 mol) triglyceryl ether. The conditions etc. are set according to those of Synthesis Example 1.

(2) Oxybutylenation

Into an autoclave, 320 g of the diketalized triglycerol and 20 g of potassium hydroxide were loaded, the air in the autoclave was replaced with dry nitrogen, and then the catalyst was completely dissolved with stirring at 140 ˚C. Subsequently, 3600 g of butylene oxide was dropwise added from a dropping apparatus, and the solution was stirred for 2 hours. Then the reaction product was removed from the autoclave, neutralized with hydrochloric acid to adjust the pH to 6 to 7, and then treated at 100 ˚C for 1 hour under reduced pressure to remove the contained water. The salt, which was produced after the treatment, was removed by filtration, to give an oxybutylenated diketalized triglycerol.

(3) Deketalization

Into a four-neck flask, 3920 g of the oxybutylenated triglycerol diketal, 70 g of 36% hydrochloric acid, and 200 g of water were loaded, deketalization was carried out at 80 ˚C for 3 hours in a sealed condition. Subsequently, the pH was adjusted to 6 to 7 with potassium hydroxide aqueous solution, and the mixture was treated at 100 ˚C for 1 hour under reduced pressure to remove the contained water. The salt, which was produced after the treatment, was removed by filtration, to give polyoxybutylene(50 mol) triglyceryl ether.

Alkylene oxide derivatives used in the present invention were prepared according to the following preparation methods of Synthesis Examples 4 to 5.

Synthesis Example 4 Polyoxyethylene(10 mol) polyoxypropylene(10 mol) dimethyl ether (block polymer)

$$CH_3O(EO)_{10}(PO)_{10}CH_3$$

Into an autoclave, 76 g of propylene glycol and 3.1 g of potassium hydroxide, as a catalyst, were loaded, the air in the autoclave was replaced with dry nitrogen, and then the catalyst was completely dissolved with stirring at 140 ˚C. Subsequently, 522g of propylene oxide was dropwise added from a dropping apparatus, and the solution was stirred for 2 hours. Then 440 g of ethylene oxide was dropwise added from a dropping apparatus, and the solution was stirred for 2 hours. Subsequently, 224 g of potassium hydroxide was loaded, the inner atmosphere of the system was replaced with dry nitrogen, and 188 g of methyl chloride was introduced under pressure at 80 to 130 ˚C. After a reaction was carried out for 5 hours, the reaction product was removed from the autoclave, neutralized with hydrochloric acid to adjust the pH to 6 to 7, and then treated at 100 ˚C for 1 hour under a reduced pressure of -0.095 MPa (50 mmHg) to remove the contained water. The salt, which was produced after the treatment, was removed by filtration to give the above-described block-type alkylene oxide derivative.
Before the reaction with methyl chloride, a sample was taken and purified. The hydroxyl value of the purified sample was 110, and the hydroxyl value of the obtained compound was 0.3. Therefore, the ratio of the number of hydrogen

atoms with respect to the number of terminal methyl groups was 0.003; thus terminal hydrogen atoms were almost completely replaced with methyl groups.

Synthesis Example 5 Polyoxyethylene(10 mol) polyoxypropylene(10 mol) dimethyl ether (random polymer)

$CH_3O[(EO)_{10}/(PO)_{10}]CH_3$

Into an autoclave, 76 g of propylene glycol and 3.1 g of potassium hydroxide, as a catalyst, were loaded, the air in the autoclave was replaced with dry nitrogen, and then the catalyst was completely dissolved with stirring at 140 ˚C. Subsequently, a mixture of 440 g of ethylene oxide and 522 g of propylene oxide was dropwise added from a dropping apparatus, and the solution was stirred for 2 hours. Subsequently, 224 g of potassium hydroxide was loaded, the inner atmosphere of the system was replaced with dry nitrogen, and 188 g of methyl chloride was introduced under pressure at 80 to 130 ˚C. After a reaction was carried out for 5 hours, the reaction product was removed from the autoclave, neutralized with hydrochloric acid to adjust the pH to 6 to 7, and then treated at 100 ˚C for 1 hour under a reduced pressure of -0.095 MPa (50 mmHg) to remove the contained water. The salt, which was produced after the treatment, was removed by filtration, to give the above-described random-type alkylene oxide derivative.
Before the reaction with methyl chloride, a sample was taken and purified. The hydroxyl value of the purified sample was 107, and the hydroxyl value of the obtained compound was 0.4. Therefore, the ratio of the number of hydrogen atoms with respect to the number of terminal methyl groups was 0.004; thus terminal hydrogen atoms were almost completely replaced with methyl groups.

I. WATER-IN-OIL TYPE EXTERNAL SKIN PREPARATIONS

Test Example I-1 Blending of the polyglycerol derivative

The present inventors initially prepared polyglycerol derivatives according to the above-described synthesis examples. A comparison was made between the water-in-oil type external skin preparations (sunscreen cream) containing the polyglycerol derivative as an emulsifier and those containing the conventional surfactant. The blending compositions and the evaluation results for the water-in-oil type external skin preparations in the respective examples and the respective comparative examples are shown in Table 1. The blending quantities are all in mass %. The evaluation criteria are as follows.

(Emulsion Stability)

The emulsion stability was evaluated for the water-in-oil type external skin preparations in the respective examples and the respective comparative examples. The evaluation was carried out by visual observation, based on the below-described three levels of criteria, immediately after the production and after allowing to stand at 50 ˚C for 2 weeks after filling into a glass bottle.

<Evaluation Criteria>

○: Emulsion state was good.
Δ: Slight separation of an oil phase or a water phase was observed.
×: Substantial separation of an oil phase or a water phase was observed.

Table 1

| | Comp. Ex. | | Example | | |
|---|---|---|---|---|---|
| | 1-1 | 1-2 | 1-1 | 1-2 | 1-3 |
| (1)Diglyceryl diisostearate | 2.0 | - | - | - | - |
| (2)POE(30mol)dipolyhydroxystearate | - | 2.0 | - | - | - |

(continued)

| | Comp. Ex. | | Example | | |
|---|---|---|---|---|---|
| | 1-1 | 1-2 | 1-1 | 1-2 | 1-3 |
| (3)POB(50mol)triglyceryl ether | - | - | 2.0 | 2.0 | 2.0 |
| (4)Glyceryl tri-2-ethylhexanoate | 25.0 | - | 25.0 | - | 25.0 |
| (5)Decamethylcyclopentasiloxane | - | 25.0 | - | 25.0 | - |
| (6)Octyl methoxycinnamate | 5.0 | 5.0 | 5.0 | 5.0 | 5.0 |
| (7)Bis-ethythexyloxyphenolmethoxytriazine | - | - | - | - | 2.0 |
| (8)Hydrophobic particulate zinc oxide*1 | - | - | - | - | 15.0 |
| (9)Purified water | Balance | Balance | Balance | Balance | Balance |
| (10)1,3-Butylene glycol | 5.0 | 5.0 | 5.0 | 5.0 | 5.0 |
| (11)Magnesium sulfate | 0.5 | 0.5 | 0.5 | 0.5 | 0.5 |
| (12)Ethanol | 5.0 | 5.0 | 5.0 | 5.0 | 5.0 |
| (13)Methylparaben | 0.2 | 0.2 | 0.2 | 0.2 | 0.2 |
| Emulsion Stability | | | | | |
| Immediately after production | × | × | ○ | ○ | ○ |
| 50˚C, 2 weeks later | × | × | ○ | ○ | ○ |
| *1: zinc oxide FINEX-25 (product of Sakai Chemical Industry Co, Ltd.) treated with silicone | | | | | |

<Preparation Method>

Raw materials (1) to (8) were dissolved and dispersed at 70 ˚C. While the dissolved and dispersed material was stirred with a disperser, (9) to (13), which had been dissolved by heating to 70 ˚C, were added. Then, the water-in-oil type external skin preparations of the respective examples and the respective comparative examples were obtained by cooling to 30 ˚C.

As is clear from Table 1, when polar oil was emulsified with diglyceryl diisostearate, which is a water-in-oil type emulsifier widely used in the past, a separation of an oil phase and a water phase was observed immediately after emulsification (Comparative Example 1-1). When silicone oil was emulsified with the use ofpolyoxyethylene(30 mol) dipolyhydroxystearate, which is suitable for the emulsification of relatively polar oil, a separation of an oil phase and a water phase was similarly observed immediately after emulsification (Comparative Example 1-2).

In contrast, when a polyglycerol derivative (polyoxybutylene(50 mol) triglyceryl ether) of the present invention was used as a water-in-oil type emulsifier, both polar oil and silicone oil could be stably-emulsified (Examples 1-1 and 1-2). In addition, a water-in-oil type emulsion product prepared by dissolving, in polar oil, an UV absorber that is a solid at an ordinary temperature and further by blending an UV scatterer was found to be also very stable (Example 1-3).

In order to further investigate the suitability of polyglycerol derivatives, the present inventors prepared various polyglycerol derivatives according to the above-described synthesis examples. Water-in-oil type external skin preparations containing these various polyglycerol derivatives were evaluated in the same way as the above-described tests. The blending compositions and the evaluation results for the water-in-oil type external skin preparations in the respective examples and the respective comparative examples are shown in Table 2.

Table 2

| | Example | | | |
|---|---|---|---|---|
| | 1-4 | 1-5 | 1-6 | 1-7 |
| (1)POB(25mol)methyl triglyceryl ether | 2.0 | — | — | — |
| (2)POB(50mol)butyl triglyceryl ether | — | 2.0 | — | — |
| (3)POB(10mol)methyl triglyceryl ether | — | — | 2.0 | — |
| (4)POB(150mol)methyl triglyceryl ether | — | — | — | 2.0 |
| (5)Triglycerin | — | — | — | — |
| (6)Methyl triglyceryl ether | — | — | — | — |
| (7)POB(50mol)hexyl triglyceryl ether | — | — | — | — |
| (8)POB(250mol)methyl triglyceryl ether | — | — | — | — |
| (9)POB(25mol)methyl triglyceryl ether*1 | — | — | — | — |
| (10)Glyceryl tri-2-ethylhexanoate | 10.0 | 10.0 | 10.0 | 10.0 |
| (11)Methylcyclopentasiloxane | 15.0 | 15.0 | 15.0 | 15.0 |
| (12)Octyl methoxycinnamate | 5.0 | 5.0 | 5.0 | 5.0 |
| (13)Purified water | Balance | Balance | Balance | Balance |
| (14)1,3-Butylene glycol | 5.0 | 5.0 | 5.0 | 5.0 |
| (15)Magnesium sulfate | 0.5 | 0.5 | 0.5 | 0.5 |
| (16)Ethanol | 5.0 | 5.0 | 5.0 | 5.0 |
| (17)Methylparaben | 0.2 | 0.2 | 0.2. | 0.2 |
| Emulsion Stability | | | | |
| Immediately after production | ○ | ○ | ○ | ○ |
| 50°C, 2 weeks later | ○ | ○ | ○ | ○ |

*1:produced according to Synthesis Example 1 without (1)ketalization and (3)deketalization.

Table 2(continued)

| | Comp. Ex. | | | | |
|---|---|---|---|---|---|
| | 1-3 | 1-4 | 1-5 | 1-6 | 1-7 |
| (1)POB(25mol)methyl triglyceryl ether | — | — | — | — | — |
| (2)POB(50mol)butyl triglyceryl ether | — | — | — | — | — |
| (3)POB(10mol)methyl triglyceryl ether | — | — | — | — | — |
| (4)POB(150mol)methyl triglyceryl ether | — | — | — | — | — |
| (5)Triglycerin | 2.0 | — | — | — | — |
| (6)Methyl triglyceryl ether | — | 2.0 | — | — | — |
| (7)POB(50mol)hexyl triglyceryl ether | — | — | 2.0 | — | — |
| (8)POB(250mol)methyl triglyceryl ether | — | — | — | 2.0 | — |
| (9)POB(25mol)methyl triglyceryl ether*1 | — | — | — | — | 2.0 |
| (10)Glyceryl tri-2-ethylhexanoate | 10.0 | 10.0 | 10.0 | 10.0 | 10.0 |
| (11)Methylcyclopentasiloxane | 15.0 | 15.0 | 15.0 | 15.0 | 15.0 |
| (12)Octyl methoxycinnamate | 5.0 | 5.0 | 5.0 | 5.0 | 5.0 |
| (13)Purified water | Balance | Balance | Balance | Balance | Balance |
| (14)1,3-Butylene glycol | 5.0 | 5.0 | 5.0 | 5.0 | 5.0 |
| (15)Magnesium sulfate | 0.5 | 0.5 | 0.5 | 0.5 | 0.5 |
| (16)Ethanol | 5.0 | 5.0 | 5.0 | 5.0 | 5.0 |
| (17)Methylparaben | 0.2 | 0.2 | 0.2 | 0.2 | 0.2 |
| Emulsion Stability | | | | | |
| Immediately after production | × | × | ○ | △ | × |
| 50°C, 2 weeks later | × | × | × | × | × |

*1:produced according to Synthesis Example 1 without (1)ketalization and (3)deketalization.

As is clear from Table 2, the water-in-oil type external skin preparations, in which the polyglycerol derivative has a methyl group or a butyl group at the terminal of the polyoxybutylene group, had excellent emulsion stability even when polar oils and silicone oils were blended (Examples 1-4 and 1-5). When a polyglycerol derivative with 10 mol or 150 mol of added oxybutylene was used, similar excellent emulsion stability was observed (Examples 1-6 and 1-7).

In contrast, when unmodified triglycerol or triglycerol modified with a methyl group was used, a separation of an oil phase and a water phase was observed immediately after emulsification (Comparative Examples 1-3 and 1-4). When a polyglycerol derivative having a hexyl group at the terminal of the polyoxybutylene group or a polyglycerol derivative with 250 mol of added oxybutylene was used, the long-term emulsion stability was confirmed to be poor (Comparative Examples 1-5 and 1-6). In addition, when a polyglycerol derivative with the polyoxybutylene group that was added without the protection of the terminal hydroxyl groups by ketalization was used, excellent emulsion stability could not be achieved (Comparative Example 1-7).

<u>Test Example I-2 Blending quantity of the polyglycerol derivative</u>

In order to investigate the desirable blending quantity of polyglycerol derivatives, the present inventors evaluated the water-in-oil type external skin preparations with varied blending quantities of polyglycerol derivatives in the same way as the above-described tests. The blending compositions and the evaluation results for the water-in-oil type external skin preparations in the respective examples and the respective comparative examples are shown in Table 3. The evaluation criteria for the feeling in use are as follows.

(Feeling in Use)

The feeling in use of the water-in-oil type external skin preparations in the respective examples and the respective comparative examples was evaluated by allowing 10 panelists, namely, five males and five females to actually use them. The evaluation was based on the below-described three levels of criteria.

<Evaluation Criteria>

○: 7 or more panelists evaluated that it is good.
Δ: 2 or more and less than 7 panelists evaluated that it is good.
×: Less than 2 panelists evaluated that it is good.

Table 3

| | Example | | | | | | |
|---|---|---|---|---|---|---|---|
| | 1-8 | 1-9 | 1-10 | 1-11 | 1-12 | 1-13 | 1-14 |
| (1)POB(25mol)methyl triglyceryl ether | 0.1 | 0.5 | 1.0 | 5.0 | 10.0 | 30.0 | 50.0 |
| (2)Glyceryl tri-2-ethylhexanoate | 10.0 | 10.0 | 10.0 | 10.0 | 10.0 | 10.0 | 10.0 |
| (3)Methylcyclopentasiloxane | 15.0 | 15.0 | 15.0 | 15.0 | 15.0 | 15.0 | 15.0 |
| (4)Octyl methoxycinnamate | 5.0 | 5.0 | 5.0 | 5.0 | 5.0 | 5.0 | 5.0 |
| (5)Purified water | Balance | Balance | Balance | Balance | Balance | Balance | Balance |
| (6)1,3-Butylene glycol | 5.0 | 5.0 | 5.0 | 5.0 | 5.0 | 5.0 | 5.0 |
| (7)Magnesium sulfate | 0.5 | 0.5 | 0.5 | 0.5 | 0.5 | 0.5 | 0.5 |
| (8)Ethanol | 5.0 | 5.0 | 5.0 | 5.0 | 5.0 | 5.0 | 5.0 |
| (9)Methylparaben | 0.2 | 0.2 | 0.2 | 0.2. | 0.2 | 0.2 | 0.2 |
| Emulsion Stability | | | | | | | |
| Immediately after Production | ○ | ○ | ○ | ○ | ○ | ○ | ○ |
| 50˚C, 2 weeks later | × | ○ | ○ | ○ | ○ | ○ | Δ |
| Feeling in use | ○ | ○ | ○ | ○ | ○ | Δ | × |

As is clear from Table 3, in the water-in-oil type external skin preparations containing polyglycerol derivatives of the present invention, if the blending quantity was 0.5 to 30.0 mass %, the emulsion stability was excellent when polar oils and silicone oils were blended (Examples 1-9 to 1-13). If the blending quantity of polyglycerol derivatives was 30.0 mass % or more, the feeling in use tends to be poor. Thus, it is preferable that the blending quantity is 10 mass % or less.

Test Example I-3 UV shielding effect

The UV shielding effect was evaluated for the water-in-oil type external skin preparation (sunscreen cream), in Example 1-3, wherein the excellent emulsion stability was observed in the above test. The test contents are as follows.

(UV Light Shielding Test)

The SPF and PFA values were measured with a high-precision in vitro SPF measurement system (refer to Japanese Unexamined Patent Publication No. H07-167781).
More specifically, a solar simulator (Solar Ultraviolet Simulator Model 600: Solar Light Co.) was used as a light source. A test sample was uniformly applied on the Transpore Tape TM (3M Co.), which was used as a base for application, at 2.0 mg/$cm^2$, and exposed to UV light. The SPF and PFA values were calculated by the arithmetic processing of transmission UV spectra. The results are shown in Table 4.
Table 4

| | Example 1-3 |
|---|---|
| SPF Value | 52 |
| PFA Value | 11 |

As is clear from Table 4, the water-in-oil type external skin preparations of the present invention (sunscreen) can achieve an excellent UV shielding effect because it has become possible to stably-blend in a large quantity of solid UV absorber and UV scatterer, which have been difficult, in the past, to stably-blend in.

In the following, the present invention will be described in further detail with reference to other examples of the water-in-oil type external skin,preparations of the present invention. In all examples described below, the evaluation results of "○" could be achieved in the above-described emulsion stability test.

| Example 2-1 Sunscreen cream | (mass%) |
|---|---|
| (A) | |
| POB(25mol)methyl triglyceryl ether | 3.0 |
| Alkyl benzoate (C12-15) | 10.0 |
| Glyceryl tri-2-ethylhexanoate | 10.0 |
| 2-Octyldodecanol | 5.0 |
| Octyl methoxycinnamate | 5.0 |
| 4-tert-Butyl-4'-methoxydibenzoylmethane | 1.0 |
| Perfume | Q.S. |
| (B) | |
| Butylene glycol | 5.0 |
| Purified water | Balance |

<Preparation method>

Component (A) was heated to 70 ˚C and dissolved. Phase (B) was heated to 70 ˚C and added to phase (A) under stirring with a disperser. The mixture was mixed well and then cooled to 30 ˚C to obtain a water-in-oil type sunscreen cream with good long-term stability and good spreadability.

| Example 2-2 Moisturizing cream | (mass%) |
|---|---|
| (A) | |
| POB(25mol)methyl triglyceryl ether | 2.0 |
| Decamethylcyclopentasiloxane | 15.0 |
| Pentaerythrityl tetraoctanoate | 5.0 |
| Glyceryl tri-2-ethylhexanoate | 5.0 |
| Petrolatum | 1.0 |
| Perfume | Q.S. |
| (B) | |
| Glycerin | 5.0 |
| Dipropylene glycol | 5.0 |
| Polyethylene glycol 6000 | 1.0 |
| L-Sodium glutamate | 1.0 |
| Purified water | Balance |

<Preparation method>

Component (A) was heated to 70 ˚C and dissolved. Phase (B) was heated to 70 ˚C and added to phase (A) under the treatment with a homomixer. The mixture was mixed well and then cooled to 30 ˚C to obtain a water-in-oil type moisturizing cream with good long-term stability and fresh light feeling in use.

II. OIL-IN-WATER TYPE EXTERNAL SKIN PREPARATIONS

Test Example II-1 Blending of the polyglycerol derivative

The present inventors have prepared various polyglycerol derivatives according to the above-described synthesis examples. A comparison was made between the oil-in-water type external skin preparations (sunscreen cream) containing the polyglycerol derivative and those containing a conventional dispersant. The blending compositions and the evaluation results for the oil-in-water type external skin preparations in the respective test examples are shown in Table 5. The blending quantities are all in mass %. The evaluation criteria are as follows.

(1) Moist Feeling after Use

The presence of a moist feeling after the use of the oil-in-water type external skin preparations in the respective examples and the respective comparative examples was evaluated with an actual use test by 10 professional panelists. The evaluation criteria are as follows.

<Evaluation Criteria>

◎: 8 or more panelists acknowledged the presence of moist feeling after use.
○: 6 or more and less than 8 panelists acknowledged the presence of moist feeling after use.
Δ: 3 or more and less than 6 panelists acknowledged the presence of moist feeling after use.
×: Less than 3 panelists acknowledged the presence of moist feeling after use.

(2) Absence of Sticky Feeling after Use

The absence of sticky feeling after the use of the oil-in-water type external skin preparations in the respective examples and the respective comparative examples was evaluated with an actual use test by 10 professional panelists. The evaluation criteria are as follows.

<Evaluation Criteria>

◎: 8 or more panelists acknowledged the absence of sticky feeling after use.
○: 6 or more and less than 8 panelists acknowledged the absence of sticky feeling after use.
Δ: 3 or more and less than 6 panelists acknowledged the absence of sticky feeling after use.
×: Less than 3 panelists acknowledged the absence of sticky feeling after use.

(3) Powder Dispersion Stability

The oil-in-water type external skin preparation in the respective examples and the respective comparative examples was put into a 50 mL sample tube (diameter: 3 cm), and the sample tube was rotated at room temperature at a speed of 45 rpm for 4 hours. The degree of powder aggregation was evaluated by visual observation. The evaluation criteria are as follows.

<Evaluation Criteria>

○: No powder aggregate was visually observed.

Δ: Some powder aggregates were visually observed.
×: Substantial powder aggregates were visually observed

(4) Emulsion Stability

The oil-in-water type external skin preparation in the respective examples and the respective comparative examples was put into a 50 mL sample tube (diameter: 3 cm), and the sample tube was rotated at room temperature at a speed of 45 rpm for 4 hours. The emulsion stability was evaluated with a microscope. The evaluation criteria are as follows.

<Evaluation Criteria>

○: No coalescence of emulsified particles was observed with a microscope.
Δ: Some coalescence of emulsified particles was observed with a microscope.
×: Coalescence of emulsified particles was observed with a microscope.

Table 5

| | Comp. Ex. | | Example | | | |
|---|---|---|---|---|---|---|
| | 3-1 | 3-2 | 3-1 | 3-2 | 3-3 | 3-4 |
| (Water phase) | | | | | | |
| Polyethylene glycol 1000 | 5.0 | 5.0 | 5.0 | 5.0 | 5.0 | 5.0 |
| Dipropylene glycol | 3.0 | 3.0 | 3.0 | 3.0 | 3.0 | 3.0 |
| PEG-60 hydrogenated caster oil | 2.0 | 2.0 | 2.0 | 2.0 | 2.0 | 2.0 |
| Sodium carboxymethylcellulose | 0.15 | 0.15 | 0.15 | 0.15 | 0.15 | 0.15 |
| Succinoglycan | 0.35 | 0.35 | 0.35 | 0.35 | 0.35 | 0.35 |
| Citric acid | Q.S. | Q.S. | Q.S. | Q.S. | Q.S. | Q.S. |
| Sodium citrate | Q.S. | Q.S. | Q.S. | Q.S. | Q.S. | Q.S. |
| EDTA-3Na·$2H_2O$ | 0.1 | 0.1 | 0.1 | 0.1 | 0.1 | 0.1 |
| Antiseptics | Q.S. | Q.S. | Q.S. | Q.S. | Q.S. | Q.S. |
| Purified water | Balance | Balance | Balance | Balance | Balance | Balance |
| (Oil phase) | | | | | | |
| Hydrophobized particulate titanium dioxide | 10.0 | 10.0 | 10.0 | 10.0 | 10.0 | 10.0 |
| Sorbitan sesquiisostearate | 1.2 | - | - | - | - | - |
| Trimethylsiloxysilicate | - | 1.2 | - | - | - | - |
| POB(25mol)methyl triglyceryl ether | - | - | 1.2 | - | - | - |
| POB(28mol)methyl triglyceryl ether | - | - | - | 1.2 | - | - |
| POB(42mol)methyl triglyceryl ether | - | - | - | - | 1.2 | - |
| POB(56mol)methyl triglyceryl ether | - | - | - | - | - | 1.2 |
| Cyclopentadimethylsiloxane | 9.0 | 9.0 | 9.0 | 9.0 | 9.0 | 9.0 |
| Octyl p-methoxycinnamate | 5.0 | 5.0 | 5.0 | 5.0 | 5.0 | 5.0 |
| Methylphenylpolysiloxane | 4.0 | 4.0 | 4.0 | 4.0 | 4.0 | 4.0 |
| Cetyl octanoate | 3.0 | 3.0 | 3.0 | 3.0 | 3.0 | 3.0 |
| (1)Moist Feeling | ○ | ○ | ◎ | ◎ | ◎ | ◎ |
| (2)No Stickiness | ○ | ○ | ◎ | ◎ | ◎ | ◎ |
| (3)Dispersion Stability | × | × | ○ | ○ | ○ | ○ |
| (4)Emulsion Stability | × | × | ○ | ○ | ○ | ○ |

As is clear from Table 5, when sorbitan sesquiisostearate or trimethylsiloxysilicate, which has been widely used as a dispersant in the past, was used in the oil-in-water type external skin preparations containing a large amount of hydrophobized particulate titanium dioxide, the dispersion stability of the hydrophobized powder and the emulsion stability were poor (Comparative Examples 3-1 and 3-2).

In contrast, when a polyglycerol derivative (polyoxybutylene(25 mol)methyl triglyceryl ether etc.) of the present invention was used as a dispersant, the feeling in use, powder dispersion stability, and emulsion stability were excellent though a large amount of hydrophobized powder was blended (Examples 3-1 to 3-4).

In order to further investigate the suitability of polyglycerol derivatives, the present inventors prepared various polyglycerol derivatives according to the above-described synthesis examples. Oil-in-water type external skin preparations containing these various polyglycerol derivatives were evaluated in the same way as the above-described tests. The blending compositions and the evaluation results for the oil-in-water type external skin preparations in the respective examples and the respective comparative examples are shown in Table 6.

Table 6

| | Example | | | |
|---|---|---|---|---|
| | 3-5 | 3-6 | 3-7 | 3-8 |
| (Water phase) | | | | |
| Polyethylene glycol 1000 | 5.0 | 5.0 | 5.0 | 5.0 |
| Dipropylene glycol | 3.0 | 3.0 | 3.0 | 3.0 |
| PEG-60 hydrogenated caster oil | 2.0 | 2.0 | 2.0 | 2.0 |
| Sodium carboxymethylcellulose | 0.15 | 0.15 | 0.15 | 0.15 |
| Succinoglycan | 0.35 | 0.35 | 0.35 | 0.35 |
| Citric acid | Q.S. | Q.S. | Q.S. | Q.S. |
| Sodium citrate | Q.S. | Q.S. | Q.S. | Q.S. |
| EDTA-3Na·$2H_2O$ | 0.1 | 0.1 | 0.1 | 0.1 |
| Antiseptics | Q.S. | Q.S. | Q.S. | Q.S. |
| Purified water | Balance | Balance | Balance | Balance |
| (Oil phase) | | | | |
| Hydrophobized particulate titanium dioxide | 10.0 | 10.0 | 10.0 | 10.0 |
| POB(50mol)triglyceryl ether | 1.2 | - | - | - |
| POB(50mol)butyl triglyceryl ether | - | 1.2 | - | - |
| POB(10mol)methyl triglyceryl ether | - | - | 1.2 | - |
| POB(150mol)methyl triglyceryl ether | - | - | - | 1.2 |
| Triglycerin | - | - | - | - |
| Methyl triglyceryl ether | - | - | - | - |
| POB(50mol)hexyl triglyceryl ether | - | - | - | - |
| POB(250mol)methyl triglyceryl ether | - | - | - | - |
| POB(25mol)methyl triglyceryl ether*1 | - | - | - | - |
| Cyclopentadimethylsiloxane | 9.0 | 9.0 | 9.0 | 9.0 |
| Octyl p-methoxycinnamate | 5.0 | 5.0 | 5.0 | 5.0 |
| Methylphenylpolysiloxane | 4.0 | 4.0 | 4.0 | 4.0 |
| Cetyl octanoate | 3.0 | 3.0 | 3.0 | 3.0 |
| (1)Moist Feeling | ◎ | ◎ | ◎ | ◎ |
| (2)No Stickiness | ◎ | ◎ | ◎ | ◎ |
| (3)Dispersion Stability | ○ | ○ | ○ | ○ |
| (4)Emulsion Stability | ○ | ○ | ○ | ○ |

*1:produced according to Synthesis Example 1 without (1)ketalization and (3)deketalization.

Table 6(continued)

| | Comp. Ex. | | | | |
|---|---|---|---|---|---|
| | 3-3 | 3-4 | 3-5 | 3-6 | 3-7 |
| (Water phase) | | | | | |
| Polyethylene glycol 1000 | 5.0 | 5.0 | 5.0 | 5.0 | 5.0 |
| Dipropylene glycol | 3.0 | 3.0 | 3.0 | 3.0 | 3.0 |
| PEG-60 hydrogenated caster oil | 2.0 | 2.0 | 2.0 | 2.0 | 2.0 |
| Sodium carboxymethylcellulose | 0.15 | 0.15 | 0.15 | 0.15 | 0.15 |
| Succinoglycan | 0.35 | 0.35 | 0.35 | 0.35 | 0.35 |
| Citric acid | Q.S. | Q.S. | Q.S. | Q.S. | Q.S. |
| Sodium citrate | Q.S. | Q.S. | Q.S. | Q.S. | Q.S. |
| EDTA-3Na·2$H_2O$ | 0.1 | 0.1 | 0.1 | 0.1 | 0.1 |
| Antiseptics | Q.S. | Q.S. | Q.S. | Q.S. | Q.S. |
| Purified water | Balance | Balance | Balance | Balance | Balance |
| (Oil phase) | | | | | |
| Hydrophobized particulate titanium dioxide | 10.0 | 10.0 | 10.0 | 10.0 | 10.0 |
| POB(50mol)triglyceryl ether | - | - | - | - | - |
| POB(50mol)butyl triglyceryl ether | - | - | - | - | - |
| POB(10mol)methyl triglyceryl ether | - | - | - | - | - |
| POB(150mol)methyl triglyceryl ether | - | - | - | - | - |
| Triglycerin | 1.2 | - | - | - | - |
| Methyl triglyceryl ether | - | 1.2 | - | - | - |
| POB(50mol)hexyl triglyceryl ether | - | - | 1.2 | - | - |
| POB(250mol)methyl triglyceryl ether | - | - | - | 1.2 | - |
| POB(25mol)methyl triglyceryl ether*1 | - | - | - | - | 1.2 |
| Cyclopentadimethylsiloxane | 9.0 | 9.0 | 9.0 | 9.0 | 9.0 |
| Octyl p-methoxycinnamate | 5.0 | 5.0 | 5.0 | 5.0 | 5.0 |
| Methylphenylpolysiloxane | 4.0 | 4.0 | 4.0 | 4.0 | 4.0 |
| Cetyl octanoate | 3.0 | 3.0 | 3.0 | 3.0 | 3.0 |
| (1)Moist Feeling | △ | △ | ◎ | ◎ | ◎ |
| (2)No Stickiness | △ | △ | ◎ | ○ | ◎ |
| (3)Dispersion Stability | × | × | × | × | × |
| (4)Emulsion Stability | × | × | × | × | × |

*1:produced according to Synthesis Example 1 without (1)ketalization and (3)deketalization.

As is clear from Table 6, oil-in-water type external skin preparations, in which the polyglycerol derivative having a hydrogen atom or a butyl group at the terminal of the polyoxybutylene group was used, had an excellent feeling in use, excellent powder dispersion stability, and excellent emulsion stability though a large amount of hydrophobized powder was blended (Examples 3-5 and 3-6). When a polyglycerol derivative with 10 mol or 150 mol of added oxybutylene was used, a similar excellent feeling in use, excellent powder dispersibility, and excellent emulsion stability were also observed (Examples 3-8 and 3-9).

In contrast, when unmodified triglycerol or triglycerol modified with a methyl group was used, the aggregation of hydrophobized powder and coalescence of emulsified particles were observed (Comparative Examples 3-3 and 3-4).

When a polyglycerol derivative having a hexyl group at the terminal of the polyoxybutylene group was used, or when a polyglycerol derivative with 250 mol of added oxybutylene was used, the powder dispersibility and emulsion stability were confirmed to be also poor (Comparative Examples 3-5 and 3-6). In addition, when a polyglycerol derivative with the polyoxybutylene group that was added without the protection of the terminal hydroxyl groups by ketalization was used, the excellent powder dispersibility and excellent emulsion stability could not be achieved (Comparative Example 2-7).

Test Example II-2 Blending quantity of the polyglycerol derivative

In order to investigate the desirable blending quantity of polyglycerol derivatives, the present inventors evaluated the oil-in-water type external skin preparations with varied blending quantities of polyglycerol derivatives in the same way as the above-described tests. The blending compositions and the evaluation results for the oil-in-water type external skin preparations in the respective examples and the respective comparative examples are shown in Table 7.

Table 7

| | Example | | | | | | |
|---|---|---|---|---|---|---|---|
| | 3-9 | 3-10 | 3-11 | 3-12 | 3-13 | 3-14 | 3-15 |
| (Water phase) | | | | | | | |
| Polyethylene glycol 1000 | 5.0 | 5.0 | 5.0 | 5.0 | 5.0 | 5.0 | 5.0 |
| Dipropylene glycol | 3.0 | 3.0 | 3.0 | 3.0 | 3.0 | 3.0 | 3.0 |
| PEG-60 hydrogenated caster oil | 2.0 | 2.0 | 2.0 | 2.0 | 2.0 | 2.0 | 2.0 |
| Sodium carboxymethyl cellulose | 0.15 | 0.15 | 0.15 | 0.15 | 0.15 | 0.15 | 0.15 |
| Succinoglycan | 0.35 | 0.35 | 0.35 | 0.35 | 0.35 | 0.35 | 0.35 |
| Citric acid | Q.S. | Q.S. | Q.S. | Q.S. | Q.S. | Q.S. | Q.S. |
| Sodium citrate | Q.S. | Q.S. | Q.S. | Q.S. | Q.S. | Q.S. | Q.S. |
| EDTA-3Na·$2H_2O$ | 0.1 | 0.1 | 0.1 | 0.1 | 0.1 | 0.1 | 0.1 |
| Antiseptics | Q.S. | Q.S. | Q.S, | Q.S. | Q.S. | Q.S. | Q.S. |
| Purified water | Balance | Balance | Balance | Balance | Balance | Balance | Balance |
| (Oil phase) | | | | | | | |
| Hydrophobized particulate titanium dioxide | 10.0 | 10.0 | 10.0 | 10.0 | 10.0 | 10.0 | 10.0 |
| POB(25mol)methyl triglyceryl ether | 0.1 | 0.2 | 0.5 | 1.0 | 3.0 | 7.0 | 9.0 |
| Cyclopentadimethyl siloxane | 9.0 | 9.0 | 9.0 | 9.0 | 9.0 | 9.0 | 9.0 |
| Octyl p-methoxy cinnamate | 5.0 | 5.0 | 5.0 | 5.0 | 5.0 | 5.0 | 5.0 |
| Methylphenylpolysiloxane | 4.0 | 4.0 | 4.0 | 4.0 | 4.0 | 4.0 | 4.0 |
| Cetyl octanoate | 3.0 | 3.0 | 3.0 | 3.0 | 3.0 | 3.0 | 3.0 |
| (1)Moist Feeling | ◎ | ◎ | ◎ | ◎ | ◎ | ◎ | ◎ |
| (2)No Stickiness | ◎ | ◎ | ◎ | ◎ | ◎ | ◎ | ○ |
| (3)Dispersion Stability | × | ○ | ○ | ○ | ○ | ○ | ○ |
| (4)Emulsion Stability | × | ○ | ○ | ○ | ○ | ○ | × |

As is clear from Table 7, especially if the blending quantity of the polyglycerol derivative is 0.2 to 7.0 mass % in the oil-in-water type external skin preparations containing polyglycerol derivatives of the present invention and a large amount of hydrophobized powder, an excellent feeling in use, excellent powder dispersion, and excellent emulsion stability were observed (Example 3-10 to 3-14). In contrast, if the blending quantity was 9.0 mass %, a phase inversion to a water-in-oil type took place and the coalescence of emulsified particles was observed (Example 3-15).

Test Example II-3 Blending of salt-tolerant thickener

In order to investigate thickening components to be blended in the oil-in-water type external skin preparations

together with polyglycerol derivatives, the present inventors evaluated oil-in-water type external skin preparations containing various thickeners with polyglycerol derivatives in the same way as the above-described tests. The blending compositions and evaluation results of the oil-in-water type external skin preparations in the respective examples and the respective comparative examples are shown in Table 8.

Table 8

| | Example | | | |
|---|---|---|---|---|
| | 3-16 | 3-17 | 3-18 | 3-19 |
| (Water phase) | | | | |
| Polyethylene glycol 1000 | 5.0 | 5.0 | 5.0 | 5.0 |
| Dipropylene glycol | 3.0 | 3.0 | 3.0 | 3.0 |
| PEG-60 hydrogenated caster oil | 2.0 | 2.0 | 2.0 | 2.0 |
| Sodium carboxymethylcellulose | 0.15 | 0.15 | 0.15 | 0.15 |
| Succinoglycan | 0.35 | - | - | - |
| Xanthane gum | - | 0.35 | - | - |
| Acrylamide*1 | - | - | 0.35 | - |
| Polyacrylic acid | - | - | - | 0.35 |
| Citric acid | Q.S. | Q.S. | Q.S. | Q.S. |
| Sodium citrate | Q.S. | Q.S. | Q.S. | Q.S. |
| EDTA-3Na·$2H_2O$ | 0.1 | 0.1 | 0.1 | 0.1 |
| Antiseptics | Q.S. | Q.S. | Q.S. | Q.S. |
| Purified water | Balance | Balance | Balance | Balance |
| (Oil phase) | | | | |
| Hydrophobized particulate titanium dioxide | 10.0 | 10.0 | 10.0 | 10.0 |
| POB(28mol)methyl triglyceryl ether | 1.2 | 1.2 | 1.2 | 1.2 |
| Cyclopentadimethylsiloxane | 9.0 | 9.0 | 9.0 | 9.0 |
| Octyl p-methoxycinnamate | 5.0 | 5.0 | 5.0 | 5.0 |
| Methylphenylpolysiloxane | 4.0 | 4.0 | 4.0 | 4.0 |
| Cetyl octanoate | 3.0 | 3.0 | 3.0 | 3.0 |
| (1)Moist Feeling | ◎ | ◎ | ◎ | ◎ |
| (2)No Stickiness | ◎ | ◎ | ◎ | ◎ |
| (3)Dispersion Stability | ○ | ○ | ○ | Δ |
| (4)Emulsion Stability | ○ | ○ | ○ | Δ |
| *1: Sepigel 305™ (product of SEPPIC) | | | | |

As seen in Table 8, when succinoglycan, xanthan gum, or acrylamide was blended as a thickener in addition to a polyglycerol derivative, the powder dispersion stability and emulsion stability were particularly excellent (Examples 3-16 to 3-18). On the other hand, when a widely used general thickener polyacrylic acid was blended, both dispersion stability and emulsion stability were poor (Example 3-19).

As for the above results, a salt is considered to have leached out over time to the water phase from the inorganic powder fine particles (titanium oxide) in the oil phase. For example, when a general thickener such as polyacrylic acid blended in Example 3-19 is used, this salt exerts a negative effect to the thickener and lowers the viscosity of the system. In contrast, when a salt-tolerant thickener such as succinoglycan etc. blended in Examples 3-16 to 3-18 is used, the salt that has leached out from the inorganic powder does not affect the thickener. As a result, the powder aggregation and the sedimentation of emulsified particles may be prevented for a long term.

<u>Test Example II-4 Blending of inulin derivative</u>

The present inventors have prepared various polyglycerol derivatives according to the above-described synthesis examples. A comparison was made between the oil-in-water type external skin preparations (sunscreen cream) containing the polyglycerol derivative and the inulin derivative and those containing a conventional dispersant. The

blending compositions and the evaluation results for the oil-in-water type external skin preparations in the respective test examples are shown in Table 9. The blending quantities are all in mass %. The evaluation criteria for "lightness in spreadability" are as follows, and others are the same as the above-described Test Example II-1.

"Lightness in Spreadability"

The lightness in spreadability after the application of the oil-in-water type external skin preparations in the respective examples and the respective comparative examples was evaluated with an actual use test by 10 professional panelists. The evaluation criteria are as follows.

<Evaluation Criteria>

◎: 8 or more panelists acknowledged that the spreadability during application was light.
○: 6 or more and less than 8 panelists acknowledged that the spreadability during application was light.
Δ: 3 or more and less than 6 panelists acknowledged that the spreadability during application was light.
×: Less than 3 panelists acknowledged that the spreadability during application was light.

Table 9

| | Comp. Ex. | | Example | | | |
|---|---|---|---|---|---|---|
| | 4-1 | 4-2 | 4-1 | 4-2 | 4-3 | 4-4 |
| (Water phase) | | | | | | |
| Polyethylene glycol 1000 | 5.0 | 5.0 | 5.0 | 5.0 | 5.0 | 5.0 |
| Dipropylene glycol | 3.0 | 3.0 | 3.0 | 3.0 | 3.0 | 3.0 |
| Inulin N-alkylurethane*1 | 1.0 | 1.0 | 1.0 | 1.0 | 1.0 | 1.0 |
| Sodium carboxymethylcellulose | 0.15 | 0.15 | 0.15 | 0.15 | 0.15 | 0.15 |
| Succinoglycan | 0.35 | 0.35 | 0.35 | 0.35 | 0.35 | 0.35 |
| Citric acid | Q.S. | Q.S. | Q.S. | Q.S. | Q.S. | Q.S. |
| Sodium citrate | Q.S. | Q.S. | Q.S. | Q.S. | Q.S. | Q.S. |
| EDTA-3Na·2$H_2O$ | 0.1 | 0.1 | 0.1 | 0.1 | 0.1 | 0.1 |
| Antiseptics | Q.S. | Q.S. | Q.S. | Q.S. | Q.S. | Q.S. |
| Purified water | Balance | Balance | Balance | Balance | Balance | Balance |
| (Oil phase) | | | | | | |
| Hydrophobized particulate titanium dioxide*2 | 5.0 | 5.0 | 5.0 | 5.0 | 5.0 | 5.0 |
| Hydrophobized particulate zinc oxide*3 | 5.0 | 5.0 | 5.0 | 5.0 | 5.0 | 5.0 |
| Sorbitan sesquiisostearate | 2.0 | - | - | - | - | - |
| Trimethylsiloxysilicate | - | 2.0 | - | - | - | - |
| POB(25mol)methyl triglyceryl ether | - | - | 2.0 | - | - | - |
| POB(28mol)methyl triglyceryl ether | - | - | - | 2.0 | - | - |
| POB(42mol)methyl triglyceryl ether | - | - | - | - | 2.0 | - |
| POB(56mol)methyl triglyceryl ether | - | - | - | - | - | 2.0 |
| Cyclopentadimethylsiloxane | 9.0 | 9.0 | 9.0 | 9.0 | 9.0 | 9.0 |
| Octyl p-methoxycinnamate | 5.0 | 5.0 | 5.0 | 5.0 | 5.0 | 5.0 |
| Methylphenyl polysiloxane | 4.0 | 4.0 | 4.0 | 4.0 | 4.0 | 4.0 |
| Cetyl octanoate | 3.0 | 3.0 | 3.0 | 3.0 | 3.0 | 3.0 |
| Lightness in Spreadability | ◎ | ◎ | ◎ | ◎ | ◎ | ◎ |
| Moist Feeling | ◎ | ◎ | ◎ | ◎ | ◎ | ◎ |

(continued)

| | | | | | | |
|---|---|---|---|---|---|---|
| (Oil phase)<br>No Stickiness<br>Dispersion Stability<br>Emulsion Stability | <br>◎<br>×<br>× | <br>◎<br>×<br>× | <br>◎<br>○<br>○ | <br>◎<br>○<br>○ | <br>◎<br>○<br>○ | <br>◎<br>○<br>○ |
| *1: INUTEC SP1 (product of ORAFTI)<br>*2:MT-100TV (product of Tayca Corporation)<br>*3:SS-Activox C80 (product of Showa Denko K.K.) | | | | | | |

As is clear from Table 9, when sorbitan sesquiisostearate or trimethylsiloxysilicate, which has been widely used as a dispersant in the past, was blended in the oil-in-water type external skin preparations containing hydrophobized particulate titanium dioxide and hydrophobized titanium dioxide, the dispersion stability of the hydrophobized powder and the emulsion stability were poor (Comparative Examples 4-1 and 4-2).

In contrast, when a polyglycerol derivative of a specific structure (polyoxybutylene(25 mol)methyl triglyceryl ether etc.) was blended together with an inulin N-alkylurethane, the feeling in use, powder dispersion stability, and emulsion stability were excellent though the two kinds of hydrophobized powders were blended (Examples 4-1 to 4-4).

In order to further investigate the suitability of polyglycerol derivatives, the present inventors prepared various polyglycerol derivatives according to the above-described synthesis examples. Oil-in-water type external skin preparations containing these various polyglycerol derivatives were evaluated in the same way as the above-described tests. The blending compositions and the evaluation results for the oil-in-water type external skin preparations in the respective examples and the respective comparative examples are shown in Table 10

Table 10

| | Example | | | |
|---|---|---|---|---|
| | 4-5 | 4-6 | 4-7 | 4-8 |
| (Water phase) | | | | |
| Polyethylene glycol 1000 | 5.0 | 5.0 | 5.0 | 5.0 |
| Dipropylene glycol | 3.0 | 3.0 | 3.0 | 3.0 |
| Inulin N-alkylurethane*1 | 1.0 | 1.0 | 1.0 | 1.0 |
| Sodium carboxymethylcellulose | 0.15 | 0.15 | 0.15 | 0.15 |
| Succinoglycan | 0.35 | 0.35 | 0.35 | 0.35 |
| Citric acid | Q.S. | Q.S. | Q.S. | Q.S. |
| Sodium citrate | Q.S. | Q.S. | Q.S. | Q.S. |
| EDTA-3Na·$2H_2O$ | 0.1 | 0.1 | 0.1 | 0.1 |
| Antiseptics | Q.S. | Q.S. | Q.S. | Q.S. |
| Purified water | Balance | Balance | Balance | Balance |
| (Oil phase) | | | | |
| Hydrophobized particulate titanium dioxide*2 | 5.0 | 5.0 | 5.0 | 5.0 |
| Hydrophobized particulate zinc oxide*3 | 5.0 | 5.0 | 5.0 | 5.0 |
| POB(50mol)triglyceryl ether | 1.2 | - | - | - |
| POB(50mol)butyltriglyceryl ether | - | 1.2 | - | - |
| POB(10mol)methyl triglyceryl ether | - | - | 1.2 | - |
| POB(150mol)methyl triglyceryl ether | - | - | - | 1.2 |
| Triglycerin | - | - | - | - |
| Methyl triglyceryl ether | - | - | - | - |
| POB(50mol)hexyl triglyceryl ether | - | - | - | - |
| POB(250mol)methyl triglyceryl ether | - | - | - | - |
| POB(25mol)methyl triglyceryl ether*4 | - | - | - | - |
| Cyclopentadimethylsiloxane | 9.0 | 9.0 | 9.0 | 9.0 |
| Octyl p-methoxycinnamate | 5.0 | 5.0 | 5.0 | 5.0 |
| Methylphenyl polysiloxane | 4.0 | 4.0 | 4.0 | 4.0 |
| Cetyl octanoate | 3.0 | 3.0 | 3.0 | 3.0 |
| Lightness in Spreadability | ◎ | ◎ | ◎ | ◎ |
| Moist Feeling | ◎ | ◎ | ◎ | ◎ |
| No Stickiness | ◎ | ◎ | ◎ | ◎ |
| Dispersion Stability | ○ | ○ | ○ | ○ |
| Emulsion Stability | ○ | ○ | ○ | ○ |

Table 10(continued)

| | Comp. Ex. | | | | |
|---|---|---|---|---|---|
| | 4-3 | 4-4 | 4-5 | 4-6 | 4-7 |
| (Water phase) | | | | | |
| Polyethylene glycol 1000 | 5.0 | 5.0 | 5.0 | 5.0 | 5.0 |
| Dipropylene glycol | 3.0 | 3.0 | 3.0 | 3.0 | 3.0 |
| Inulin N-alkylurethane*1 | 1.0 | 1.0 | 1.0 | 1.0 | 1.0 |
| Sodium carboxymethylcellulose | 0.15 | 0.15 | 0.15 | 0.15 | 0.15 |
| Succinoglycan | 0.35 | 0.35 | 0.35 | 0.35 | 0.35 |
| Citric acid | Q.S. | Q.S. | Q.S. | Q.S. | Q.S. |
| Sodium citrate | Q.S. | Q.S. | Q.S. | Q.S. | Q.S. |
| EDTA-3Na·$2H_2O$ | 0.1 | 0.1 | 0.1 | 0.1 | 0.1 |
| Antiseptics | Q.S. | Q.S. | Q.S. | Q.S. | Q.S. |
| Purified water | Balance | Balance | Balance | Balance | Balance |
| (Oil phase) | | | | | |
| Hydrophobized particulate titanium dioxide*2 | 5.0 | 5.0 | 5.0 | 5.0 | 5.0 |
| Hydrophobized particulate zinc oxide*3 | 5.0 | 5.0 | 5.0 | 5.0 | 5.0 |
| POB(50mol)triglyceryl ether | - | - | - | - | - |
| POB(50mol)butyltriglyceryl ether | - | - | - | - | - |
| POB(10mol)methyl triglyceryl ether | - | - | - | - | - |
| POB(150mol)methyl triglyceryl ether | - | - | - | - | - |
| Triglycerin | 1.2 | - | - | - | - |
| Methyl triglyceryl ether | - | 1.2 | - | - | - |
| POB(50mol)hexyl triglyceryl ether | - | - | 1.2 | - | - |
| POB(250mol)methyl triglyceryl ether | - | - | - | 1.2 | - |
| POB(25mol)methyl triglyceryl ether*4 | - | - | - | - | 1.2 |
| Cyclopentadimethylsiloxane | 9.0 | 9.0 | 9.0 | 9.0 | 9.0 |
| Octyl p-methoxycinnamate | 5.0 | 5.0 | 5.0 | 5.0 | 5.0 |
| Methylphenyl polysiloxane | 4.0 | 4.0 | 4.0 | 4.0 | 4.0 |
| Cetyl octanoate | 3.0 | 3.0 | 3.0 | 3.0 | 3.0 |
| Lightness in Spreadability | △ | △ | ○ | ○ | ○ |
| Moist Feeling | △ | △ | ◎ | ◎ | ◎ |
| No Stickiness | △ | △ | ◎ | ○ | ◎ |
| Dispersion Stability | × | × | × | × | × |
| Emulsion Stability | × | × | × | × | × |

*1: INUTEC SP1(product of ORAFTI)
*2:MT-100TV (product of Tayca Corporation)
*3:SS-Activox C80 (product of Showa Denko K.K.)

*4: produced according to Synthesis Example 1 without (1)ketalization and (3)deketalization.

As is clear from Table 10, oil-in-water type external skin preparations, in which the polyglycerol derivative having a hydrogen atom or a butyl group at the terminal of the polyoxybutylene group was used, had an excellent feeling

in use, excellent powder dispersion stability, and excellent emulsion stability though a large amount of hydrophobized powder was blended (Examples 4-5 and 4-6). When a polyglycerol derivative with 10 mol or 150 mol of added oxybutylene was used, a similar excellent feeling in use, excellent powder dispersibility, and excellent emulsion stability were also observed (Examples 4-7 and 4-8).

In contrast, when unmodified triglycerol or triglycerol modified with a methyl group was used, the aggregation of hydrophobized powder and coalescence of emulsified particles were observed (Comparative Examples 4-3 and 4-4). When a polyglycerol derivative having a hexyl group at the terminal of the polyoxybutylene group was used, or when a polyglycerol derivative with 250 mol of added oxybutylene was used, the powder dispersibility and emulsion stability were confirmed to also be poor (Comparative Examples 4-5 and 4-6). In addition, when a polyglycerol derivative with the polyoxybutylene group that was added without the protection of the terminal hydroxyl groups by ketalization was used, the excellent powder dispersibility or excellent emulsion stability could not be achieved (Comparative Example 4-7).

In order to investigate the suitability of an emulsifier that is blended together with a polyglycerol derivative, the present inventors evaluated the oil-in-water type external skin preparations with various emulsifiers in the same way as the above-described tests. The blending compositions and the evaluation results for the oil-in-water type external skin preparations in the respective examples and the respective comparative examples are shown in Table 11.

Table 11

| | Example | Comp. Ex. | |
|---|---|---|---|
| | 4-2 | 4-8 | 4-9 |
| (Water phase) | | | |
| Polyethylene glycol 1000 | 5.0 | 5.0 | 5.0 |
| Dipropylene glycol | 3.0 | 3.0 | 3,0 |
| Inulin N-alkylurethane*1 | 3.0 | - | - |
| POE(15)POP(15)glycol | - | 3.0 | - |
| PEG-60 hydrogenated caster oil | - | - | 3.0 |
| Sodium carboxymethylcellulose | 0.15 | 0.15 | 0.15 |
| Succinoglycan | 0.35 | 0.35 | 0.35 |
| Citric acid | Q.S. | Q.S. | Q.S. |
| Sodium citrate | Q.S. | Q.S. | Q.S. |
| EDTA-3Na·2$H_2$O | 0.1 | 0.1 | 0.1 |
| Antiseptics | Q.S. | Q.S. | Q.S. |
| Purified water | Balance | Balance | Balance |
| (Oil phase) | | | |
| Hydrophobized particulate titanium dioxide*2 | 5.0 | 5.0 | 5.0 |
| Hydrophobized particulate zinc oxide*3 | 5.0 | 5.0 | 5.0 |
| POB(28mol)methyl triglyceryl ether | 2.0 | 2.0 | 2.0 |
| Cyclopentadimethylsiloxane | 9.0 | 9.0 | 9.0 |
| Octyl p-methoxycinnamate | 5.0 | 5.0 | 5.0 |
| Methylphenyl polysiloxane | 4.0 | 4.0 | 4.0 |
| Cetyl octanoate | 3.0 | 3.0 | 3.0 |
| Lightness in Spreadability | ◎ | ◎ | ◎ |
| Moist Feeling | ◎ | Δ | Δ |
| No Stickiness | ◎ | Δ | Δ |
| Dispersion Stability | ○ | × | × |
| Emulsion Stability | ○ | × | × |
| *1: INUTEC SP1 (product of ORAFTI)<br>*2:MT-100TV (product of Tayca Corporation)<br>*3:SS-Activox C80 (product of Showa Denko K.K.) | | | |

As is clear from Table 11, the oil-in-water type external skin preparations containing an inulin N-alkylurethane

as an emulsifier together with a polyglycerol derivative had excellent evaluation results not only in the feeling in use but also both in the powder dispersion stability and emulsion stability (Example 4-2). In contrast, the oil-in-water type external skin preparations containing polyoxyethylene(15) polyoxypropylene(15) glycol or PEG-60 hydrogenated castor oil as an emulsifier could not achieve an excellent feeling in use though a polyglycerol derivative was blended. In addition, the aggregation of hydrophobized powder and coalescence of emulsified particles were observed (Comparative Examples 4-8 and 4-9).

In order to investigate thickening components that are blended in the oil-in-water type external skin preparations, the present inventors evaluated oil-in-water type external skin preparations containing various thickeners in the same way as the above-described tests. The blending compositions and evaluation results of the oil-in-water type external skin preparations in the respective examples and the respective comparative examples are shown in Table 12.

Table 12

| | Example | | | |
|---|---|---|---|---|
| | 4-9 | 4-10 | 4-11 | 4-12 |
| (Water phase) | | | | |
| Polyethylene glycol 1000 | 5.0 | 5.0 | 5.0 | 5.0 |
| Dipropylene glycol | 3.0 | 3.0 | 3.0 | 3.0 |
| Inulin N-alkylurethane*1 | 1.0 | 1.0 | 1.0 | 1.0 |
| Sodium carboxymethylcellulose | 0.15 | 0.15 | 0.15 | 0.15 |
| Succinoglycan | 0.35 | - | - | - |
| Xanthane gum | - | 0.35 | - | - |
| Acrylamide*2 | - | - | 0.35 | - |
| Polyacrylic acid | - | - | - | 0.35 |
| Citric acid | Q.S. | Q.S. | Q.S. | Q.S. |
| Sodium citrate | Q.S. | Q.S. | Q.S. | Q.S. |
| EDTA-3Na·2$H_2O$ | 0.1 | 0.1 | 0.1 | 0.1 |
| Antiseptics | Q.S. | Q.S. | Q.S. | Q.S. |
| Purified water | Balance | Balance | Balance | Balance |
| (Oil phase) | | | | |
| Hydrophobized particulate titanium dioxide*3 | 5.0 | 5.0 | 5.0 | 5.0 |
| Hydrophobized particulate zinc oxide*4 | 5.0 | 5.0 | 5.0 | 5.0 |
| POB(28mol) methyl triglyceryl ether | 3.0 | 3.0 | 3.0 | 3.0 |
| Cyclopentadimethylsiloxane | 9.0 | 9.0 | 9.0 | 9.0 |
| Octyl p-methoxycinnamate | 5.0 | 5.0 | 5.0 | 5.0 |
| Methylphenyl polysiloxane | 4.0 | 4.0 | 4.0 | 4.0 |
| Cetyl octanoate | 3.0 | 3.0 | 3.0 | 3.0 |
| Lightness in Spreadability | ◎ | ◎ | ◎ | ◎ |
| Moist Feeling | ◎ | ◎ | ◎ | ◎ |
| No Stickiness | ◎ | ◎ | ◎ | ◎ |
| Dispersion Stability | ○ | ○ | ○ | Δ |
| Emulsion Stability | ○ | ○ | ○ | Δ |
| *1: INUTEC SP1 (product of ORAFTI)<br>*2: Sepigel 305™ (product of SEPPIC)<br>*3:MT-100TV (product of Tayca Corporation)<br>*4:SS-Activox C80 (product of Showa Denko K.K.) | | | | |

As seen in Table 12, when succinoglycan, xanthan gum, or acrylamide was blended as a thickener in addition to a polyglycerol derivative and an inulin derivative, the powder dispersion stability and emulsion stability were particularly excellent (Examples 4-9 to 4-11). On the other hand, when a widely used general thickener polyacrylic acid was blended, both dispersion stability and emulsion stability were poor (Example 4-12).

As for the above results, a salt is considered to have leached out over time to the water phase from the inorganic powder fine particles (titanium oxide) in the oil phase. For example, when a general thickener such as polyacrylic acid blended in Example 4-12 is used, this salt exerts a negative effect to the thickener and lowers the viscosity of the system. In contrast, when a salt-tolerant thickener such as succinoglycan etc. blended in Examples 4-9 to 4-11 is used, the salt that has leached out from the inorganic powder does not affect the thickener. As a result, the powder aggregation and the sedimentation of emulsified particles may be prevented for a long term.

Test Example II-5 Blending of block-type alkylene oxide derivative

The present inventors have prepared various polyglycerol derivatives and block-type alkylene oxide derivatives according to the above-described synthesis examples. A comparison was made between the oil-in-water type external skin preparations (sunscreen cream) containing the polyglycerol derivative and the block-type alkylene oxide derivative and those containing a conventional dispersant. The blending compositions and the evaluation results for the oil-in-water type external skin preparations in the respective test examples are shown in Table 13. The blending quantities are all in mass %. The evaluation criteria are the same as those described in Test Examples II-1 and II-4.

Table 13

| | Comp. Ex. | | Example | | | |
|---|---|---|---|---|---|---|
| | 5-1 | 5-2 | 5-1 | 5-2 | 5-3 | 5-4 |
| (Water phase) | | | | | | |
| Polyethylene glycol 1000 | 5.0 | 5.0 | 5.0 | 5.0 | 5.0 | 5.0 |
| Dipropylene glycol | 3.0 | 3.0 | 3.0 | 3.0 | 3.0 | 3.0 |
| $CH_3O(EO)_{35}(PO)_{40}CH_3$(block) | 1.0 | 1.0 | 1.0 | 1.0 | 1.0 | 1.0 |
| Sodium carboxymethylcellulose | 0.15 | 0,15 | 0.15 | 0.15 | 0.15 | 0.15 |
| Succinoglycan | 0.35 | 0.35 | 0.35 | 0.35 | 0.35 | 0.35 |
| Citric acid | Q.S. | Q.S. | Q.S. | Q.S. | Q.S. | Q.S. |
| Sodium citrate | Q.S. | Q.S. | Q.S. | Q.S, | Q.S. | Q.S. |
| EDTA-3Na·$2H_2O$ | 0.1 | 0.1 | 0.1 | 0.1 | 0.1 | 0.1 |
| Antiseptics | Q.S. | Q.S. | Q.S. | Q.S, | Q.S. | Q.S. |
| Purified water | Balance | Balance | Balance | Balance | Balance | Balance |
| (Oil phase) | | | | | | |
| Hydrophobized particulate titanium dioxide*1 | 5.0 | 5.0 | 5.0 | 5.0 | 5.0 | 5.0 |
| Hydrophobized particulate zinc oxide*2 | 5.0 | 5.0 | 5.0 | 5.0 | 5.0 | 5.0 |
| Sorbitan sesquiisostearate | 2.0 | - | - | - | - | - |
| Trimethylsiloxysilicate | - | 2.0 | - | - | - | - |
| POB(25mol)methyl triglyceryl ether | - | - | 2.0 | - | - | - |
| POB(28mol)methyl triglyceryl ether | - | - | - | 2.0 | - | - |
| POB(42mol)methyl triglyceryl ether | - | - | - | - | 2.0 | - |
| POB(56mol)methyl triglyceryl ether | - | - | - | - | - | 2.0 |
| Cyclopentadimethylsiloxane | 9.0 | 9.0 | 9.0 | 9.0 | 9.0 | 9.0 |
| Octyl p-methoxycinnamate | 5.0 | 5.0 | 5.0 | 5.0 | 5.0 | 5.0 |
| Methylphenyl polysiloxane | 4.0 | 4.0 | 4.0 | 4.0 | 4.0 | 4.0 |
| Cetyl octanoate | 3.0 | 3.0 | 3.0 | 3.0 | 3.0 | 3.0 |
| Lightness in Spreadability | ◎ | ◎ | ◎ | ◎ | ◎ | ◎ |
| Moist Feeling | ◎ | ◎ | ◎ | ◎ | ◎ | ◎ |
| No Stickiness | ◎ | ◎ | ◎ | ◎ | ◎ | ◎ |
| Dispersion Stability | × | × | ○ | ○ | ○ | ○ |

(continued)

| | | | | | | |
|---|---|---|---|---|---|---|
| (Oil phase)<br>Emulsion Stability | × | × | ○ | ○ | ○ | ○ |
| *1:MT-100TV (product of Tayca Corporation)<br>*2:SS-Activox C80 (product of Showa Denko K.K.) | | | | | | |

As is clear from Table 13, when sorbitan sesquiisostearate or trimethylsiloxysilicate, which has been widely used as a dispersant in the past, was blended in the oil-in-water type external skin preparations containing hydrophobized particulate titanium dioxide and hydrophobized titanium dioxide, the dispersion stability of the hydrophobized powder and the emulsion stability were poor (Comparative Examples 5-1 and 5-2).

In contrast, when a polyglycerol derivative of a specific structure (polyoxybutylene(25 mol)methyl triglyceryl ether etc.) was blended together with a block-type alkylene oxide derivative, the feeling in use, powder dispersion stability, and emulsion stability were excellent though the two kinds of hydrophobized powders were blended (Examples 5-1 to 5-4).

In order to further investigate the suitability of polyglycerol derivatives, the present inventors prepared various polyglycerol derivatives according to the above-described synthesis examples. Oil-in-water type external skin preparations containing these various polyglycerol derivatives were evaluated in the same way as the above-described tests. The blending compositions and the evaluation results for the oil-in-water type external skin preparations in the respective examples and the respective comparative examples are shown in Table 14.

Table 14

| | Example | | | |
|---|---|---|---|---|
| | 5-5 | 5-6 | 5-7 | 5-8 |
| (Water phase) | | | | |
| Polyethylene glycol 1000 | 5.0 | 5.0 | 5.0 | 5.0 |
| Dipropylene glycol | 3.0 | 3.0 | 3.0 | 3.0 |
| $CH_3O(EO)_{35}(PO)_{40}CH_3$(block) | 1.0 | 1.0 | 1.0 | 1.0 |
| Sodium carboxymethylcellulose | 0.15 | 0.15 | 0.15 | 0.15 |
| Succinoglycan | 0.35 | 0.35 | 0.35 | 0.35 |
| Citric acid | Q.S. | Q.S. | Q.S. | Q.S. |
| Sodium citrate | Q.S. | Q.S. | Q.S. | Q.S. |
| EDTA-3Na·$2H_2O$ | 0.1 | 0.1 | 0.1 | 0.1 |
| Antiseptics | Q.S. | Q.S. | Q.S. | Q.S. |
| Purified water | Balance | Balance | Balance | Balance |
| (Oil phase) | | | | |
| Hydrophobized particulate titanium dioxide*1 | 5.0 | 5.0 | 5.0 | 5.0 |
| Hydrophobized particulate zinc oxide*2 | 5.0 | 5.0 | 5.0 | 5.0 |
| POB(50mol)triglyceryl ether | 1.2 | - | - | - |
| POB(50mol)butyltriglyceryl ether | - | 1.2 | - | - |
| POB(10mol)methyl triglyceryl ether | - | - | 1.2 | - |
| POB(150mol)methyl triglyceryl ether | - | - | - | 1.2 |
| Triglycerin | - | - | - | - |
| Methyl triglyceryl ether | - | - | - | - |
| POB(50mol)hexyl triglyceryl ether | - | - | - | - |
| POB(250mol)methyl triglyceryl ether | - | - | - | - |
| POB(25mol)methyl triglyceryl ether*3 | - | - | - | - |
| Cyclopentadimethylsiloxane | 9.0 | 9.0 | 9.0 | 9.0 |
| Octyl p-methoxycinnamate | 5.0 | 5.0 | 5.0 | 5.0 |
| Methylphenyl polysiloxane | 4.0 | 4.0 | 4.0 | 4.0 |
| Cetyl octanoate | 3.0 | 3.0 | 3.0 | 3.0 |
| Lightness in Spreadability | ◎ | ◎ | ◎ | ◎ |
| Moist Feeling | ◎ | ◎ | ◎ | ◎ |
| No Stickiness | ◎ | ◎ | ◎ | ◎ |
| Dispersion Stability | ○ | ○ | ○ | ○ |
| Emulsion Stability | ○ | ○ | ○ | ○ |

Table 14(continued)

| | Comp. Ex. | | | | |
|---|---|---|---|---|---|
| | 5-3 | 5-4 | 5-5 | 5-6 | 5-7 |
| (Water phase) | | | | | |
| Polyethylene glycol 1000 | 5.0 | 5.0 | 5.0 | 5.0 | 5.0 |
| Dipropylene glycol | 3.0 | 3.0 | 3.0 | 3.0 | 3.0 |
| $CH_3O(EO)_{35}(PO)_{40}CH_3$(block) | 1.0 | 1.0 | 1.0 | 1.0 | 1.0 |
| Sodium carboxymethylcellulose | 0.15 | 0.15 | 0.15 | 0.15 | 0.15 |
| Succinoglycan | 0.35 | 0.35 | 0.35 | 0.35 | 0.35 |
| Citric acid | Q.S. | Q.S. | Q.S. | Q.S. | Q.S. |
| Sodium citrate | Q.S. | Q.S. | Q.S. | Q.S. | Q.S. |
| EDTA-3Na·$2H_2O$ | 0.1 | 0.1 | 0.1 | 0.1 | 0.1 |
| Antiseptics | Q.S. | Q.S. | Q.S. | Q.S. | Q.S. |
| Purified water | Balance | Balance | Balance | Balance | Balance |
| (Oil phase) | | | | | |
| Hydrophobized particulate titanium dioxide*1 | 5.0 | 5.0 | 5.0 | 5.0 | 5.0 |
| Hydrophobized particulate zinc oxide*2 | 5.0 | 5.0 | 5.0 | 5.0 | 5.0 |
| POB(50mol)triglyceryl ether | - | - | - | - | - |
| POB(50mol)butyltriglyceryl ether | - | - | - | - | - |
| POB(10mol)methyl triglyceryl ether | - | - | - | - | - |
| POB(150mol)methyl triglyceryl ether | - | - | - | - | - |
| Triglycerin | 1.2 | - | - | - | - |
| Methyl triglyceryl ether | - | 1.2 | - | - | - |
| POB(50mol)hexyl triglyceryl ether | - | - | 1.2 | - | - |
| POB(250mol)methyl triglyceryl ether | - | - | - | 1.2 | - |
| POB(25mol)methyl triglyceryl ether*3 | - | - | - | - | 1.2 |
| Cyclopentadimethylsiloxane | 9.0 | 9.0 | 9.0 | 9.0 | 9.0 |
| Octyl p-methoxycinnamate | 5.0 | 5.0 | 5.0 | 5.0 | 5.0 |
| Methylphenyl polysiloxane | 4.0 | 4.0 | 4.0 | 4.0 | 4.0 |
| Cetyl octanoate | 3.0 | 3.0 | 3.0 | 3.0 | 3.0 |
| Lightness in Spreadability | △ | △ | ○ | ○ | ○ |
| Moist Feeling | △ | △ | ◎ | ◎ | ◎ |
| No Stickiness | △ | △ | ◎ | ○ | ◎ |
| Dispersion Stability | × | × | × | × | × |
| Emulsion Stability | × | × | × | × | × |

*1:MT-100TV (product of Tayca Corporation)

*2:SS-Activox C80 (product of Showa Denko K.K.)

*3: produced according to Synthesis Example 1 without (1)ketalization and (3)deketalization.

As is clear from Table 14, oil-in-water type external skin preparations, in which the polyglycerol derivative

having a hydrogen atom or a butyl group at the terminal of the polyoxybutylene group was used, had an excellent feeling in use, excellent powder dispersion stability, and excellent emulsion stability though a large amount of hydrophobized powder was blended (Examples 5-5 and 5-6). When a polyglycerol derivative with 10 mol or 150 mol of added oxybutylene was used, a similar excellent feeling in use, excellent powder dispersibility, and excellent emulsion stability were also observed (Examples 5-7 and 5-8).

In contrast, when unmodified triglycerol or triglycerol modified with a methyl group was used, the aggregation of hydrophobized powder and coalescence of emulsified particles were observed (Comparative Examples 5-3 and 5-4). When a polyglycerol derivative having a hexyl group at the terminal of the polyoxybutylene group was used, or when a polyglycerol derivative with 250 mol of added oxybutylene was used, the powder dispersibility and emulsion stability were confirmed to also be poor (Comparative Examples 5-5 and 5-6). In addition, when a polyglycerol derivative with the polyoxybutylene group that was added without the protection of the terminal hydroxyl groups by ketalization was used, the excellent powder dispersibility or excellent emulsion stability could not be achieved (Comparative Example 5-7).

In order to further investigate the suitability of an alkylene oxide derivative that is blended together with a polyglycerol derivative, the present inventors prepared various alkylene oxide derivatives according to the above-described synthesis examples. The oil-in-water type external skin preparations containing various alkylene oxide derivatives were evaluated in the same way as the above-described tests. The blending compositions and evaluation results of the oil-in-water type external skin preparations in the respective examples and the respective comparative examples are shown in Table 15.

Table 15

| | Example | | Comp. Ex. | | |
|---|---|---|---|---|---|
| | 5-9 | 5-10 | 5-8 | 5-9 | 5-10 |
| (Water phase) | | | | | |
| Polyethylene glycol 1000 | 5.0 | 5.0 | 5.0 | 5.0 | 5.0 |
| Dipropylene glycol | 3.0 | 3.0 | 3.0 | 3.0 | 3.0 |
| $CH_3O(EO)_{25}(PO)_{30}CH_3$(block) | 3.0 | - | - | - | - |
| $CH_3O(EO)_{50}(PO)_{40}CH_3$(block) | - | 3.0 | - | - | - |
| $CH_3O(EO)_{30}CH_3$ | - | - | 3.0 | - | - |
| $CH_3O(PO)_{30}CH_3$ | - | - | - | 3.0 | - |
| $CH_3O[(EO)_{36}(PO)_{41}]CH_3$(random) | - | - | - | - | 3.0 |
| $CH_3O(EO)_9(PO)_2CH_3$(block) | - | - | - | - | - |
| $CH_3O(EO)_{80}(PO)_{40}CH_3$(block) | - | - | - | - | - |
| $HO(EO)_{15}(PO)_{15}H$(block) | - | - | - | - | - |
| $C_6H_{13}O(EO)_{15}(PO)_{15}C_6H_{13}$(block) | - | - | - | - | - |
| Sodium carboxymethylcellulose | 0.15 | 0.15 | 0.15 | 0.15 | 0.15 |
| Succinoglycan | 0.35 | 0.35 | 0.35 | 0.35 | 0.35 |
| Citric acid | Q.S. | Q.S. | Q.S. | Q.S. | Q.S. |
| Sodium citrate | Q.S. | Q.S. | Q.S. | Q.S. | Q.S. |
| EDTA-3Na·$2H_2O$ | 0.1 | 0.1 | 0.1 | 0.1 | 0.1 |
| Antiseptics | Q.S. | Q.S. | Q.S. | Q.S. | Q.S. |
| Purified water | Balance | Balance | Balance | Balance | Balance |
| (Oil phase) | | | | | |
| Hydrophobized particulate titanium dioxide*1 | 5.0 | 5.0 | 5.0 | 5.0 | 5.0 |
| Hydrophobized particulate zinc oxide*2 | 5.0 | 5.0 | 5.0 | 5.0 | 5.0 |
| POB(28mol)methyl triglyceryl ether | 2.0 | 2.0 | 2.0 | 2.0 | 2.0 |
| Cyclopentadimethylsiloxane | 9.0 | 9.0 | 9.0 | 9.0 | 9.0 |
| Octyl p-methoxycinnamate | 5.0 | 5.0 | 5.0 | 5.0 | 5.0 |
| Methylphenyl polysiloxane | 4.0 | 4.0 | 4.0 | 4.0 | 4.0 |
| Cetyl octanoate | 3.0 | 3.0 | 3.0 | 3.0 | 3.0 |
| Lightness in Spreadability | ◎ | ◎ | △ | ◎ | ◎ |
| Moist Feeling | ◎ | ◎ | ◎ | △ | ◎ |
| No Stickiness | ◎ | ◎ | × | ◎ | ◎ |
| Dispersion Stability | ○ | ○ | × | × | × |
| Emulsion Stability | ○ | ○ | × | × | × |

Table 15(continued)

| | Comp. Ex.. | | | |
|---|---|---|---|---|
| | 5-11 | 5-12 | 5-13 | 5-14 |
| (Water phase) | | | | |
| Polyethylene glycol 1000 | 5.0 | 5.0 | 5.0 | 5.0 |
| Dipropylene glycol | 3.0 | 3.0 | 3.0 | 3.0 |
| $CH_3O(EO)_{25}(PO)_{30}CH_3$(block) | - | - | - | - |
| $CH_3O(EO)_{50}(PO)_{40}CH_3$(block) | - | - | - | - |
| $CH_3O(EO)_{30}CH_3$ | - | - | - | - |
| $CH_3O(PO)_{30}CH_3$ | - | - | - | - |
| $CH_3O[(EO)_{36}(PO)_{41}]CH_3$(random) | - | - | - | - |
| $CH_3O(EO)_9(PO)_2CH_3$(block) | 3.0 | - | - | - |
| $CH_3O(EO)_{80}(PO)_{40}CH_3$(block) | - | 3.0 | - | - |
| $HO(EO)_{15}(PO)_{15}H$(block) | - | - | 3.0 | - |
| $C_6H_{13}O(EO)_{15}(PO)_{15}C_6H_{13}$(block) | - | - | - | 3.0 |
| Sodium carboxymethylcellulose | 0.15 | 0.15 | 0.15 | 0.15 |
| Succinoglycan | 0.35 | 0.35 | 0.35 | 0.35 |
| Citric acid | Q.S. | Q.S. | Q.S. | Q.S. |
| Sodium citrate | Q.S. | Q.S. | Q.S. | Q.S. |
| EDTA-3Na·$2H_2O$ | 0.1 | 0.1 | 0.1 | 0.1 |
| Antiseptics | Q.S. | Q.S. | Q.S. | Q.S. |
| Purified water | Balance | Balance | Balance | Balance |
| (Oil phase) | | | | |
| Hydrophobized particulate titanium dioxide*1 | 5.0 | 5.0 | 5.0 | 5.0 |
| Hydrophobized particulate zinc oxide*2 | 5.0 | 5.0 | 5.0 | 5.0 |
| POB(28mol)methyl triglyceryl ether | 2.0 | 2.0 | 2.0 | 2.0 |
| Cyclopentadimethylsiloxane | 9.0 | 9.0 | 9.0 | 9.0 |
| Octyl p-methoxycinnamate | 5.0 | 5.0 | 5.0 | 5.0 |
| Methylphenyl polysiloxane | 4.0 | 4.0 | 4.0 | 4.0 |
| Cetyl octanoate | 3.0 | 3.0 | 3.0 | 3.0 |
| Lightness in Spreadability | ◎ | △ | △ | ◎ |
| Moist Feeling | ◎ | ◎ | ◎ | △ |
| No Stickiness | ◎ | × | △ | △ |
| Dispersion Stability | × | ○ | ○ | × |
| Emulsion Stability | × | ○ | ○ | × |

*1:MT-100TV (product of Tayca Corporation)
*2:SS-Activox C80 (product of Showa Denko K.K.)

As is clear from Table 15, for the oil-in-water type external skin preparations containing a block-type alkylene

oxide derivative represented by formula (2-b) (for example, $CH_3O(EO)_{25}(PO)_{30}CH_3$ block polymer etc.) as an emulsifier together with a polyglycerol derivative, excellent evaluation results were obtained not only in the feeling in use but also both in the powder dispersion stability and emulsion stability (Examples 5-9 and 5-10).

In contrast, when an alkylene oxide derivative with only oxyethylene groups or only oxypropylene groups was used, the feeling in use was poor and the powder dispersion stability and emulsion stability were low (Comparative Examples 5-8 and 5-9). When a random alkylene oxide derivative or an alkylene oxide derivative with the molecular weight of less than 1000 was used, the powder dispersion stability and emulsion stability were poor (Comparative Examples 5-10 and 5-11). When an alkylene oxide derivative having 80 mol or more of oxyethylene group or an alkylene oxide derivative having a hydroxyl group at both terminals was used, the feeling in use was not desirable (Comparative Examples 5-12 and 5-13). When an alkylene oxide derivative with a hydrocarbon group having 6 carbon atoms at both terminals was used, the dispersion stability and emulsion stability were not satisfactory (Comparative Example 5-14),

In order to investigate thickening components that are blended in the oil-in-water type external skin preparations, the present inventors evaluated oil-in-water type external skin preparations containing various thickeners in the same way as the above-described tests. The blending compositions and evaluation results of the oil-in-water type external skin preparations in the respective examples and the respective comparative examples are shown in Table 16.

Table 16

| | Example | | | |
|---|---|---|---|---|
| | 5-11 | 5-12 | 5-13 | 5-14 |
| (Water phase) | | | | |
| Polyethylene glycol 1000 | 5.0 | 5.0 | 5.0 | 5.0 |
| Dipropylene glycol | 3.0 | 3.0 | 3.0 | 3.0 |
| $CH_3O(EO)_{35}(PO)_{40}CH_3$(block) | 1.0 | 1.0 | 1.0 | 1.0 |
| Sodium carboxymethylcellulose | 0.15 | 0.15 | 0.15 | 0.15 |
| Succinoglycan | 0.35 | - | - | - |
| Xanthane gum | - | 0.35 | - | - |
| Acrylamide*1 | - | - | 0.35 | - |
| Polyacrylic acid | - | - | - | 0.35 |
| Citric acid | Q.S. | Q.S. | Q.S. | Q.S. |
| Sodium citrate | Q.S. | Q.S. | Q.S. | Q.S. |
| EDTA-3Na·$2H_2O$ | 0.1 | 0.1 | 0.1 | 0.1 |
| Antiseptics | Q.S. | Q.S. | Q.S. | Q.S. |
| Purified water | Balance | Balance | Balance | Balance |
| (Oil phase) | | | | |
| Hydrophobized particulate titanium dioxide*2 | 5.0 | 5.0 | 5.0 | 5.0 |
| Hydrophobized particulate zinc oxide*3 | 5.0 | 5.0 | 5.0 | 5.0 |
| POB(28mo1)methyl triglyceryl ether | 3.0 | 3.0 | 3.0 | 3.0 |
| Cyclopentadimethylsiloxane | 9.0 | 9.0 | 9.0 | 9.0 |
| Octyl p-methoxycinnamate | 5.0 | 5.0 | 5.0 | 5.0 |
| Methylphenyl polysiloxane | 4.0 | 4.0 | 4.0 | 4.0 |
| Cetyl octanoate | 3.0 | 3.0 | 3.0 | 3.0 |
| Lightness in Spreadability | ◎ | ◎ | ◎ | ◎ |
| Moist Feeling | ◎ | ◎ | ◎ | ◎ |
| No Stickiness | ◎ | ◎ | ◎ | ◎ |
| Dispersion Stability | ○ | ○ | ○ | Δ |

(continued)

| | | | | |
|---|---|---|---|---|
| (Oil phase)<br>Emulsion Stability | ○ | ○ | ○ | Δ |
| *1: Sepigel 305™ (product of SEPPIC)<br>*2:MT-100TV (product of Tayca Corporation)<br>*3:SS-Activox C80 (product of Showa Denko K.K.) | | | | |

As seen in Table 16, when succinoglycan, xanthan gum, or acrylamide was blended as a thickener in addition to a polyglycerol derivative and a block-type alkylene oxide derivative, the powder dispersion stability and emulsion stability were particularly excellent (Examples 5-11 to 5-13). On the other hand, when a widely used general thickener polyacrylic acid was blended, both dispersion stability and emulsion stability were poor (Example 5-14).

As for the above results, a salt is considered to have leached out over time to the water phase from the inorganic powder fine particles (titanium oxide) in the oil phase. For example, when a general thickener such as polyacrylic acid blended in Example 5-14 is used, this salt exerts a negative effect to the thickener and lowers the viscosity of the system. In contrast, when a salt-tolerant thickener such as succinoglycan etc. blended in Examples 5-11 to 5-13 is used, the salt that has leached out from the inorganic powder does not affect the thickener. As a result, the powder aggregation and the sedimentation of emulsified particles may be prevented for a long term.

| Example 6-1: Oil-in-water type sun-cut milky lotion | (mass%) |
|---|---|
| (1)Hydrophobized particulate titanium dioxide | 12 |
| (2)POB(42mol)triglyceryl ether | 1.5 |
| (3)Decamethylpentacyclosiloxane | 10 |
| (4)Octyl p-methoxycinnamate | 5 |
| (5)Glyceryl tri-2-ethylhexanoate | 3 |
| (6)PEG-60 hydrogenated caster oil | 2 |
| (7)1,3-Butylene glycol | 8 |
| (8)Succinoglycan | 0.2 |
| (9)Carboxymethylcellulose | 0.25 |
| (10)Ethanol | 3 |
| (11)Ion-exchanged water | Balance |

<Preparation method>

The mixture of (1)-(5) was dispersed and crushed with a bead mill, and then added into the water phase, which was obtained by dissolving (6)-(11), while being treated with a homomixer.

| Example 6-2: Oil-in-water type emulsified liquid foundation | (mass%) |
|---|---|
| (1)Hydrophobized particulate titanium dioxide | 15 |
| (2)Hydrophobized iron oxide yellow | 0.7 |
| (3)Hydrophobized iron oxide black | 0.18 |
| (4)Hydrophobized iron oxide red | 0.32 |
| (5)POB(28mol)triglyceryl ether | 2 |
| (6)Decamethylpentacyclosiloxane | 10 |
| (7)Octyl p-methoxycinnamate | 5 |
| (8)Octyldodecyl myristate | 3 |
| (9)PEG-60 hydrogenated caster oil | 2 |
| (10)Dynamite glycerin | 4 |
| (11)Xanthane gum | 0.3 |
| (12)Carboxymethylcellulose | 0.3 |
| (13)Ethanol | 5 |

(continued)

| Example 6-2: Oil-in-water type emulsified liquid foundation (mass%) | |
|---|---|
| (14)Ion-exchanged water | Balance |

<Preparation method>

The mixture of (1)-(8) was dispersed and crushed with a bead mill, and then added into the water phase, which was obtained by dissolving (9)-(14), while being treated with a homomixer.

| Example 6-3: UV-protecting whitening essence | (mass%) |
|---|---|
| (1)Hydrophobized particulate titanium dioxide (silicone-treated) | 20 |
| (2)POB(56mol)triglyceryl ether | 2.5 |
| (3)Decamethylpentacyclosiloxane | 10 |
| (4)Octyl p-methoxycinnamate | 5 |
| (5)Isopropyl palmitate | 4 |
| (6)PEG-60 hydrogenated caster oil | 2 |
| (7)Dynamite glycerin | 5 |
| (8)Succinoglycan | 0.3 |
| (9)Carboxymethylcellulose | 0.3 |
| (10)Ethanol | 4 |
| (11)Citric acid | Q.S. |
| (12) Sodium citrate | Q.S. |
| (13)Ascorbyl glucoside | 2 |
| (14)Potassium hydroxide | Q.S. |
| (15)Ion-exchanged water | Balance |

<Preparation method>

The mixture of (1)-(5) was dispersed and crushed with a bead mill, and then added into the water phase, which was obtained by dissolving (6)-(15), while being treated with a homomixer.

All of the oil-in-water type cosmetics, shown in Examples 6-1 to 6-3, provided a good feeling in use. In particular, there was a moist feeling after use without sticky feeling. In addition, the powder dispersion stability and the emulsion stability were excellent.

| Example 6-4: Oil-in-water type sun-cut milky lotion | (mass%) |
|---|---|
| (1)Hydrophobized particulate titanium dioxide | 3 |
| (2)Hydrophobized particulate zinc oxide | 7 |
| (3)POB(42mol)triglyceryl ether | 2 |
| (4)Decamethylcyclopentasiloxane | 10 |
| (5)Octyl p-methoxycinnamate | 5 |
| (6)Glyceryl tri-2-ethylhexanoate | 3 |
| (7)Inulin N-alkylurethane | 2 |
| (INUTEC SP1: product of ORAFTI) | |
| (8)1,3-Butylene glycol | 8 |
| (9)Succinoglycan | 0.2 |
| (10)Carboxymethylcellulose | 0.25 |
| (11)Ethanol | 3 |
| (12)Ion-exchanged water | Balance |

<Preparation method>

The mixture of (1)-(6) was dispersed and crushed with a bead mill, and then added into the water phase, which was obtained by dissolving (7)-(12), while being treated with a homomixer.

| Example 6-5: Oil-in-water type sun-cut milky lotion | (mass%) |
|---|---|
| (1)Hydrophobized particulate titanium dioxide | 12 |
| (2)POB(28mol)triglyceryl ether | 2.5 |
| (3)Decamethylpentacyclosiloxane | 8 |
| (4)Octyl p-methoxycinnamate | 7 |
| (5)Glyceryl tri-2-ethylhexanoate | 5 |
| (6)Inulin N-alkylurethane | 1 |
| (INUTEC SP1: product of ORAFTI) | |
| (7)Dynamite glycerin | 6 |
| (8) Succinoglycan | 0.3 |
| (9)Carboxymethylcellulose | 0.25 |
| (10)Ethanol | 2.5 |
| (11)Ion-exchanged water | Balance |

<Preparation method>

The mixture of (1)-(5) was dispersed and crushed with a bead mill, and then added into the water phase, which was obtained by dissolving (6)-(11), while being treated with a homomixer.

| Example 6-6: Oil-in-water type sun-cut milky lotion | (mass%) |
|---|---|
| (1)Hydrophobized particulate zinc oxide | 15 |
| (2)POB(56mol)triglyceryl ether | 3 |
| (3)Decamethylpentacyclosiloxane | 12 |
| (4)Octyl p-methoxycinnamate | 7 |
| (5)Glyceryl tri-2-ethylhexanoate | 4 |
| (6)Inulin N-alkylurethane | 3 |
| (INUTEC SP1: product of ORAFTI) | |
| (7)1,3-Butylene glycol | 8 |
| (8) Succinoglycan | 0.3 |
| (9)Carboxymethylcellulose | 0.2 |
| (10) Ethanol | 2 |
| (11) Ion-exchanged water | Balance |

<Preparation method>

The mixture of (1)-(5) was dispersed and crushed with a bead mill, and then added into the water phase, which was obtained by dissolving (6)-(11), while being treated with a homomixer.

| Example 6-7: Oil-in-water type emulsified liquid foundation | (mass%) |
|---|---|
| (1)Hydrophobized particulate titanium dioxide | 7 |
| (2)Hydrophobized particulate zinc oxide | 9 |
| (3)Hydrophobized iron oxide yellow | 0.8 |
| (4)Hydrophobized iron oxide black | 0.16 |
| (5)Hydrophobized iron oxide red | 0.36 |

(continued)

| Example 6-7: Oil-in-water type emulsified liquid foundation | (mass%) |
|---|---|
| (6)POB(56mol)methyl triglyceryl ether | 3 |
| (7)Decamethylpentacyclosiloxane | 10 |
| (8)Octyl p-methoxycinnamate | 5 |
| (9)Octyldodecyl myristate | 3 |
| (10)INUTEC SP1 | 1.5 |
| (11)Dynamite glycerin | 4 |
| (12)Xanthane gum | 0.3 |
| (13)Carboxymethylcellulose | 0.3 |
| (14)Ethanol | 5 |
| (15)Ion-exchanged water | Balance |

<Preparation method>

The mixture of (1)-(9) was dispersed and crushed with a bead mill, and then added into the water phase, which was obtained by dissolving (10)-(15), while being treated with a homomixer.

| Example 6-8: UV-protecting whitening essence | (mass%) |
|---|---|
| (1)Hydrophobized particulate titanium dioxide (silicone-treated) | 6 |
| (2)Hydrophobized particulate zinc oxide | 8 |
| (3)POB(42mol)methyl triglyceryl ether | 2.8 |
| (4)Decamethylpentacyclosiloxane | 10 |
| (5)Octyl p-methoxycixinamate | 5 |
| (6)Isopropyl palmitate | 4 |
| (7)INUTEC SP1 | 2.5 |
| (8)Dynamite glycerin | 5 |
| (9)Succinoglycan | 0.3 |
| (10)Carboxymethylcellulose | 0.3 |
| (11)Ethanol | 4 |
| (12)Citric acid | Q.S. |
| (13)Sodium citrate | Q.S. |
| (14)Ascorbyl glucoside | 2 |
| (15)Potassium hydroxide | Q.S. |
| (16)Ion-exchanged water | Balance |

<Preparation method>

The mixture of (1)-(6) was dispersed and crushed with a bead mill, and then added into the water phase, which was obtained by dissolving (7)-(16), while being treated with a homomixer.

| Example 6-9: Oil-in-water type sun-cut milky lotion | (mass%) |
|---|---|
| (1)Hydrophobized particulate titanium dioxide | 3 |
| (2)Hydrophobized particulate zinc oxide | 7 |
| (3)POB(42mol)triglyceryl ether | 2 |
| (4)Decamethylpentacyclosiloxane | 10 |
| (5)Octyl p-methoxycinnamate | 5 |
| (6)Glyceryl tri-2-ethylhexanoate | 3 |
| (7)$CH_3O(EO)_{35}(PO)_{40}CH_3$(block polymer) | 2 |
| (8)1,3-Butylene glycol | 8 |

(continued)

| Example 6-9: Oil-in-water type sun-cut milky lotion | (mass%) |
|---|---|
| (9)Succinoglycan | 0.2 |
| (10)Carboxymethylcellulose | 0.25 |
| (11)Ethanol | 3 |
| (12)Ion-exchanged water | Balance |

<Preparation method>

The mixture of (1)-(6) was dispersed and crushed with a bead mill, and then added into the water phase, which was obtained by dissolving (7)-(12), while being treated with a homomixer.

| Example 6-10: Oil-in-water type sun-cut milky lotion | (mass%) |
|---|---|
| (1)Hydrophobized particulate titanium dioxide | 12 |
| (2)POB(28mol)triglyceryl ether | 2.5 |
| (3)Decamethylpentacyclosiloxane | 8 |
| (4)Octyl p-methoxycinnamate | 7 |
| (5)Glyceryl tri-2-ethylhexanoate | 5 |
| (6)$CH_3O(EO)_{35}(PO)_{40}CH_3$(block polymer) | 3 |
| (7)Dynamite glycerin | 6 |
| (8)Succinoglycan | 0.3 |
| (9)Carboxymethylcellulose | 0.25 |
| (10)Ethanol | 2.5 |
| (11)Ion-exchanged water | Balance |

<Preparation method>

The mixture of (1)-(5) was dispersed and crushed with a bead mill, and then added into the water phase, which was obtained by dissolving (6)-(11), while being treated with a homomixer.

| Example 6-11:Oil-in-water type sun-cut milky lotion | (mass%) |
|---|---|
| (1)Hydrophobized particulate zinc oxide | 15 |
| (2)POB(56mol)triglyceryl ether | 3 |
| (3)Decamethylpentacyclosiloxane | 12 |
| (4)Octyl p-methoxycinnamate | 7 |
| (5)Glyceryl tri-2-ethylhexanoate | 4 |
| (6)$CH_3O(EO)_{35}(PO)_{40}CH_3$(block polymer) | 4 |
| (7)1,3-Butylene glycol | 8 |
| (8)Succinoglycan | 0.3 |
| (9)Carboxymethylcellulose | 0.2 |
| (10)Ethanol | 2 |
| (11) Ion-exchanged water | Balance |

<Preparation method>

The mixture of (1)-(5) was dispersed and crushed with a bead mill, and then added into the water phase, which was obtained by dissolving (6)-(11), while being treated with a homomixer.

| Example 6-12: Oil-in-water type emulsified liquid foundation | (mass%) |
|---|---|
| (1)Hydrophobized particulate titanium dioxide | 4 |
| (2)Hydrophobized particulate zinc oxide | 6 |
| (3)Hydrophobized iron oxide yellow | 0.8 |
| (4)Hydrophobized iron oxide black | 0.16 |
| (5)Hydrophobized iron oxide red | 0.36 |
| (6)POB(56mol)methyl triglyceryl ether | 3 |
| (7)Decamethylpentacyclosiloxane | 10 |
| (8)Octyl p-methoxycinnamate | 5 |
| (9)Octyldodecyl myristate | 3 |
| (10)$CH_3O(EO)_{35}(PO)_{40}CH_3$(block polymer) | 2 |
| (11)Dynamite glycerin | 4 |
| (12)Xanthane gum | 0.3 |
| (13)Carboxymethylcellulose | 0.3 |
| (14)Ethanol | 5 |
| (15)Ion-exchanged water | Balance |

<Preparation method>

The mixture of (1)-(9) was dispersed and crushed with a bead mill, and then added into the water phase, which was obtained by dissolving (10)-(15), while being treated with a homomixer.

| Example 6-13: UV-protecting whitening essence | (mass%) |
|---|---|
| (1)Hydrophobized particulate titanium dioxide (silicone-treated) | 5 |
| (2)Hydrophobized particulate zinc oxide | 5 |
| (3)POB(42mol)methyl triglyceryl ether | 2.5 |
| (4)Decamethylpentacyclosiloxane | 10 |
| (5)Octyl p-methoxycinnamate | 5 |
| (6)Isopropyl palmitate | 4 |
| (7)$CH_3O(EO)_{35}(PO)_{40}CH_3$(block polymer) | 2 |
| (8)Dynamite glycerin | 5 |
| (9)Succinoglycan | 0.3 |
| (10)Carboxymethylcellulose | 0.3 |
| (11)Ethanol | 4 |
| (12)Citric acid | Q.S. |
| (13)Sodium citrate | Q.S. |
| (14)Ascorbyl glucoside | 2 |
| (15)Potassium hydroxide | Q.S. |
| (16)Ion-exchanged water | Balance |

<Preparation method>

The mixture of (1)-(6) was dispersed and crushed with a bead mill, and then added into the water phase, which was obtained by dissolving (7)-(16), while being treated with a homomixer.

All of the oil-in-water type cosmetics, shown in Examples 6-4 to 6-13, provided a good feeling in use. In particular, the spreadability during the application was light, and there was a moist feeling after use without sticky feeling. In addition, the dispersion stability and the emulsion stability were excellent.

## Claims

**1.** An emulsified external skin preparation comprising a polyglycerol derivative represented by formula (1):

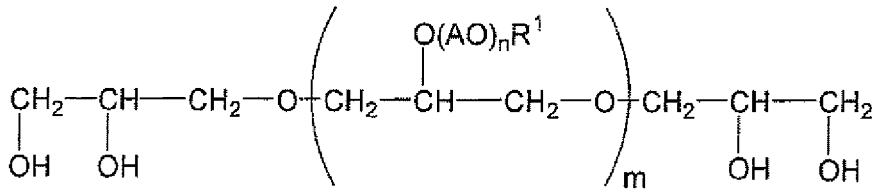

(1)

wherein, m + 2 represents the average polymerization degree of polyglycerol and $1 \leqq m \leqq 4$;

$R^1$ is a hydrocarbon group having 1 to 4 carbon atoms or a hydrogen atom;
AO is an oxyalkylene group having 3 to 4 carbon atoms; and
n is the average addition mole number of the oxyalkylene group and $1 \leqq m \times n \leqq 200$

**2.** The emulsified external skin preparation according to claim 1, wherein the preparation is a water-in-oil type emulsified external skin preparation.

**3.** The water-in-oil type emulsified external skin preparation according to claim 2, further comprising a polar oil.

**4.** The water-in-oil type emulsified external skin preparation according to claim 3, wherein the polar oil is one or more selected from the group consisting of polar oils with an IOB value of 0.05 to 0.80.

**5.** The water-in-oil type emulsified external skin preparation according to claim 4, wherein the polar oil is one or more selected from the group consisting of 2-ethylhexyl p-methoxycinnamate, 2-ethylhexyl 2-cyano-3,3-diphenylacrylate, tripropylene glycol dipivalate, cetyl octanoate, trimethylolpropane tri-2-ethylhexanoate, pentaerythritol tetra-2-ethylhexanoate, glyceryl tri-2-ethylhexanoate, $C_{12-15}$ alkyl benzoate, caprylic/capric triglyceride, propylene glycol dicaprylate/dicaprate, and di-2-ethylhexyl succinate.

**6.** The water-in-oil type emulsified external skin preparation according to any of claims 1 to 5, further comprising a silicone oil.

**7.** The water-in-oil type emulsified external skin preparation according to any of claims 1-6, further comprising an UV absorber that is a solid at ordinary temperature.

**8.** The water-in-oil type emulsified external skin preparation according to claim 7, the UV absorber that is a solid at ordinary temperature is selected from the group consisting of 2,4-bis-[[4-(2-ethylhexyloxy)-2-hydroxy]-phenyl]-6-(4-methoxyphenyl)-1,3,5-triazine, 2,4,6-trianilino-(p-carbo-2'-ethylhexyl-1'-oxy)-1,3,5-triazine, and 1-[4-(1,1-dimethylethyl) phenyl]-3-(4-methoxyphenyl)-1,3-propanedione.

**9.** The water-in-oil type emulsified external skin preparation according to any of claims 1-8, further comprising an UV scatterer.

**10.** The water-in-oil type emulsified external skin preparation according to any of claims 1-9, wherein the preparation is an external skin preparation for sunscreen that is used for sun-protecting.

**11.** The emulsified external skin preparation according to claim 1, wherein the preparation is an oil-in-water type emulsified external skin preparation.

**12.** The oil-in-water type emulsified external skin preparation according to claim 11, further comprising hydrophobized powder.

**13.** The oil-in-water type emulsified external skin preparation according to claim 12, wherein the hydrophobized powder is hydrophobized particulate titanium dioxide and/or hydrophobized particulate zinc oxide.

**14.** The oil-in-water type emulsified external skin preparation according to claim 12 or 13, further comprising an inulin derivative represented by formula (2-a).

$$A\text{-}(O\text{-}CO\text{-}NH\text{-}R^1)s \qquad (2\text{-}a)$$

wherein A is a fructose residue of inulin;

(O-CO-NH-$R^1$) represents a N-alkylaminocarbonyloxy group substituting a hydroxyl group of the fructose;
$R^1$ is a hydrocarbon group having 3 to 22 carbon atoms; and
s is the substitution degree of the N-alkylaminocarbonyloxy group per fructose residue, and s takes 0.10 to 2.0.

**15.** The oil-in-water type emulsified external skin preparation according to claim 12 or 13, further comprising a block-type alkylene oxide derivative represented by formula (2-b):

$$R^1O\text{-}(AO)_m(EO)_n\text{-}R^2 \qquad (2\text{-}b)$$

wherein AO is an oxyalkylene group having 3 to 4 carbon atoms;

EO is an oxyethylene group;
m and n are average addition mole numbers of the oxyalkylene group and the oxyethylene group respectively, which are $1 \leqq m \leqq 70$, $1 \leqq n \leqq 70$;
the oxyalkylene group having 3 to 4 carbon atoms and the oxyethylene group are added to each other in block form;
the oxyethylene group is 20 to 80 mass % with respect to the sum of the oxyalkylene group having 3 to 4 carbon atoms and the oxyethylene group; and
$R^1$ and $R^2$ are either identical to or different from each other, and they are either a hydrocarbon group having 1 to 4 carbon atoms or hydrogen atom, and wherein the ratio of the number of the hydrogen atom with respect to the number of the hydrocarbon group in $R^1$ and $R^2$ is 0.15 or less.

**16.** The oil-in-water type emulsified external skin preparation according to claim 14 or 15, wherein the preparation comprises the hydrophobized particulate titanium dioxide and hydrophobized particulate zinc oxide as the hydrophobized powder.

**17.** The oil-in-water type emulsified external skin preparation according to any of claims 11 to 16, further comprising one or more selected from the group consisting of succinoglycan, xanthan gum, and acrylamide.

**18.** The oil-in-water type emulsified external skin preparation according to any of claims 11 to 17, further comprising one or more selected from the group consisting of carboxymethylcellulose, hydroxyethyl cellulose, hydroxymethyl cellulose, and gelatin.

**19.** The oil-in-water type emulsified external skin preparation according to any of claims 11 to 18, wherein the preparation is an external skin preparation for sunscreen that is used for sun-protecting.

**INTERNATIONAL SEARCH REPORT**

International application No. PCT/JP2006/314721

A. CLASSIFICATION OF SUBJECT MATTER
*A61K8/86*(2006.01)i, *A61K8/06*(2006.01)i, *A61Q17/04*(2006.01)i

According to International Patent Classification (IPC) or to both national classification and IPC

B. FIELDS SEARCHED

Minimum documentation searched (classification system followed by classification symbols)
A61K8/86, A61K8/06, A61Q17/04

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2006 |
| Kokai Jitsuyo Shinan Koho | 1971-2006 | Toroku Jitsuyo Shinan Koho | 1994-2006 |

Electronic data base consulted during the international search (name of data base and, where practicable, search terms used)
CAplus/MEDLINE/BIOSIS/EMBASE/REGISTRY(STN)

C. DOCUMENTS CONSIDERED TO BE RELEVANT

| Category* | Citation of document, with indication, where appropriate, of the relevant passages | Relevant to claim No. |
|---|---|---|
| A | JP 2005-089752 A (CLARIANT GMBH), 07 April, 2005 (07.04.05), & DE 10342870 A1 & EP 1518900 A2 & US 2005/112081 A1 | 1-19 |
| A | WO 92/08685 A1 (OREAL), 29 May, 1992 (29.05.92), & AT 117665 A & CA 2073353 A1 & DE 69107056 D1 & DE 69107056 A & DK 510165 A & EP 0510165 A1 & ES 2067255 A & FR 2669023 A1 & FR 2680171 A1 & GR 3015846 A & JP 05-505623 A & US 5362494 A & US 5539129 A | 1-19 |

☒ Further documents are listed in the continuation of Box C.  ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 28 September, 2006 (28.09.06) | 10 October, 2006 (10.10.06) |
| Name and mailing address of the ISA/ Japanese Patent Office | Authorized officer |
| Facsimile No. | Telephone No. |

Form PCT/ISA/210 (second sheet) (April 2005)

**INTERNATIONAL SEARCH REPORT**

International application No.
PCT/JP2006/314721

C (Continuation). DOCUMENTS CONSIDERED TO BE RELEVANT

| Category* | Citation of document, with indication, where appropriate, of the relevant passages | Relevant to claim No. |
|---|---|---|
| A | JP 05-097628 A (GOLDWELL AG),<br>20 April, 1993 (20.04.93),<br>& AT 89716T T & CA 2056892 A1<br>& DE 4100490 C1 & DE 69100094 T2<br>& DK 494391T T3 & EP 0494391 A1<br>& ES 2047369T T3 & US 5213799 A | 1-19 |
| A | JP 49-019046 A (Shiseido Co., Ltd.),<br>20 February, 1974 (20.02.74),<br>(Family: none) | 1-19 |
| A | JP 09-302087 A (NOF Corp.),<br>25 November, 1997 (25.11.97),<br>(Family: none) | 1-19 |

Form PCT/ISA/210 (continuation of second sheet) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2005216492 A
- JP 2005216493 A
- JP 2005232553 A
- JP 2005232554 A
- JP H10501252 W
- JP H1112125 B
- JP H0794366 B
- JP H08310940 B
- JP H07167781 B


### Non-patent literature cited in the description


- **FUJITA.** *Kagaku No Ryoiki,* 1957, vol. 11 (10), 719-725